# EUROPEAN PATENT SPECIFICATION

(11) **EP 1 594 537 B1**
(45) Date of publication and mention of the grant of the patent: **24.10.2012**
(21) Application number: 03815962.0
(22) Date of filing: 19.02.2003
(51) Int. Cl.: A61K 39/12, A61K 39/245, A61K 39/155, C12N 15/09, C12N 15/33, C12N 15/38, C12N 15/40, C12N 15/45, A61P 11/00

(54) **VACCINATION OR IMMUNIZATION USING A PRIME-BOOST REGIMEN AGAINST BRSV, BHV-1, BVDV, BPI-3**
VAKZINIERUNG ODER IMMUNISIERUNG GEGEN BRSV, BHV-1,BVDV,BPI-3, DIE EIN PRIME-BOOST-VERFAHREN VERWENDET
VACCINATION OU IMMUNISATION UTILISANT UN SCHEMA POSOLOGIQUE DE DOUBLE ADMINISTRATION D'AMOR AGE ET DE RAPPEL CONTRE LE VRS, LE VIRUS-HERPES BOVIN 1, LE VIRUS BVD, LE VIRUS DE PARAINFLUENZA BOVIN DE TYPE 3

(43) Date of publication of application: 16.11.2005
(73) Proprietor: MERIAL, 69007 Lyon (FR)
(72) Inventor: AUDONNET, Jean-Christophe, Francis, F-69002 Lyon (FR); FISCHER, Laurent, Bernard, F-69110 Sainte Foy Les Lyon (FR); BARZU-LE-ROUX, Simona, F-69210 Lentilly (FR)
(74) Representative: Harding, Charles Thomas
(86) International application number: PCT/IB2003/001215
(87) International publication number: WO 2004/073737

(56) References cited:
- WO-A-01/52888
- WO-A-92/07940
- LOEHR B I ET AL: "Priming by DNA immunization augments T-cell responses induced by modified live bovine herpesvirus vaccine" JOURNAL OF GENERAL VIROLOGY, vol. 82, no. 12, December 2001 (2001-12), pages 3035-3043, XP002256791 ISSN: 0022-1317
- BABIUK SHAWN ET AL: "Electroporation improves the efficacy of DNA vaccines in large animals" VACCINE, vol. 20, no. 27-28, 10 September 2002 (2002-09-10), pages 3399-3408, XP004378535 ISSN: 0264-410X
- RAMSHAW I A ET AL: "The prime-boost strategy: exciting prospects for improved vaccination" IMMUNOLOGY TODAY, ELSEVIER PUBLICATIONS, CAMBRIDGE, GB, vol. 21, no. 4, April 2000 (2000-04), pages 163-165, XP004194963 ISSN: 0167-5699
- SCHRIJVER R S ET AL: "Immunization of cattle with a BHV1 vector vaccine or a DNA vaccine both coding for the G protein of BRSV", VACCINE, ELSEVIER LTD, GB, vol. 15, no. 17-18, 1 December 1997 (1997-12-01), pages 1908-1916, XP004097385, ISSN: 0264-410X, DOI: DOI:10.1016/S0264-410X(97)00129-1
- TAYLOR G ET AL: "Role of T-lymphocyte subsets in recovery from respiratory syncytial virus infection in calves", JOURNAL OF VIROLOGY, vol. 69, no. 11, 1995, pages 6658-6664, ISSN: 0022-538X
- ELLIS JOHN ET AL: "Efficacy of an inactivated respiratory syncytial virus vaccine in calves", JOURNAL OF THE AMERICAN VETERINARY MEDICAL ASSOCIATION, AMERICAN VETERINARY MEDICAL ASSOCIATION, US, vol. 218, no. 12, 15 June 2001 (2001-06-15), pages 1973-1980, XP009111367, ISSN: 0003-1488, DOI: DOI:10.2460/JAVMA.2001.218.1973

### SEQUENCE LISTING

<110> Merial
<120> Vaccination or immunization using a prime-boost regimen
<130> Prime-boost bovine
<160> 59
<170> PatentIn version 3.2
<210> 1
   <211> 40
   <212> DNA
   <213> Artificial
<220>
   <223> oligonucleotide as primer for PCR
<400> 1
   gatctgcagc acgtgtctag aggatatcga attcgcggcc 40
<210> 2
   <211> 40
   <212> DNA
   <213> Artificial
<220>
   <223> oligonucleotide as primer for PCR
<400> 2
   gatccgcggc cgcgaattcg atatcctcta gacacgtgct 40
<210> 3
   <211> 20
   <212> DNA
   <213> Artificial
<220>
   <223> oligonucleotide as primer for PCR
<400> 3
   ttggggaccc ttgattgttc 20
<210> 4
   <211> 21
   <212> DNA
   <213> Artificial
<220>
   <223> oligonucleotide as primer for PCR
<400> 4
   ctgtaggaaa aagaagaagg c 21
<210> 5
   <211> 30
   <212> DNA
   <213> Artificial
<220>
   <223> oligonucleotide as primer for PCR
<400> 5
   ctccatgtcg acttggggac ccttgattgt 30
<210> 6
   <211> 30
   <212> DNA
   <213> Artificial
<220>
   <223> oligonucleotide as primer for PCR
<400> 6
   ctccatgtcg acctgtagga aaaagaagaa 30
<210> 7
   <211> 30
   <212> DNA
   <213> Artificial
<220>
   <223> oligonucleotide as primer for PCR
<400> 7
   ttgtcgacat ggccgctcgc ggcggtgctg 30
<210> 8
   <211> 21
   <212> DNA
   <213> Artificial
<220>
   <223> oligonucleotide as primer for PCR
<400> 8
   gcagggcagc ggctagcgcg g 21
<210> 9
   <211> 52
   <212> DNA
   <213> Artificial
<220>
   <223> oligonucleotide as primer for PCR
<400> 9
   ctgcacgagc tccggttcta cgacattgac cgcgtggtca agacggactg ag 52
<210> 10
   <211> 56
   <212> DNA
   <213> Artificial
<220>
   <223> oligonucleotide as primer for PCR
<400> 10
   gatcctcagt ccgtcttgac cacgcggtca atgtcgtaga accggagctc gtgcag 56
<210> 11
   <211> 39
   <212> DNA
   <213> Artificial
<220>
   <223> oligonucleotide as primer for PCR
<400> 11
   aaaatttcga tatccgccgc ggggcgaccg gcgacaacg 39
<210> 12
   <211> 33
   <212> DNA
   <213> Artificial
<220>
   <223> oligonucleotide as primer for PCR
<400> 12
   ggaagatctt cagtccgtct tgaccacgcg gtc 33
<210> 13
   <211> 37
   <212> DNA
   <213> Artificial
<220>
   <223> oligonucleotide as primer for PCR
<400> 13
   tcgtgcctgc ggcgcaaggc ccgggcgcgc ctgtagt 37
<210> 14
   <211> 37
   <212> DNA
   <213> Artificial
<220>
   <223> oligonucleotide as primer for PCR
<400> 14
   ctagactaca ggcgcgcccg ggccttgcgc cgcaggc 37
<210> 15
   <211> 43
   <212> DNA
   <213> Artificial
<220>
   <223> oligonucleotide as primer for PCR
<400> 15
   gcaccgctgc ccgagttctc cgcgaccgcc acgtacgact agt 43
<210> 16
   <211> 43
   <212> DNA
   <213> Artificial
<220>
   <223> oligonucleotide as primer for PCR
<400> 16
   ctagactagt cgtacgtggc ggtcgcggag aactcgggca gcg 43
<210> 17
   <211> 39
   <212> DNA
   <213> Artificial
<220>
   <223> oligonucleotide as primer for PCR
<400> 17
   aaaatttcga tatcccggcg ggggctcgcc gaggaggcg 39
<210> 18
   <211> 32
   <212> DNA
   <213> Artificial
<220>
   <223> oligonucleotide as primer for PCR
<400> 18
   ggaagatctc tagtcgtacg tggcggtcgc gg 32
<210> 19
   <211> 33
   <212> DNA
   <213> Artificial
<220>
   <223> oligonucleotide as primer for PCR
<400> 19
   tttctgcaga tgcaagggcc gacattggcc gtg 33
<210> 20
   <211> 31
   <212> DNA
   <213> Artificial
<220>
   <223> oligonucleotide as primer for PCR
<400> 20
   tttctagatt agggcgtagc gggggcgggc g 31
<210> 21
   <211> 39
   <212> DNA
   <213> Artificial
<220>
   <223> oligonucleotide as primer for PCR
<400> 21
   aaaatttcga tatcccccgc gccgcgggtg acggtatac 39
<210> 22
   <211> 33
   <212> DNA
   <213> Artificial
<220>
   <223> oligonucleotide as primer for PCR
<400> 22
   ggaagatctt tagggcgtag cgggggcggg cgg 33
<210> 23
   <211> 34
   <212> DNA
   <213> Artificial
<220>
   <223> oligonucleotide as primer for PCR
<400> 23
   aaattttctg cagatggcga caacagccat gagg 34
<210> 24
   <211> 35
   <212> DNA
   <213> Artificial
<220>
   <223> oligonucleotide as primer for PCR
<400> 24
   ttaaggatcc tcatttacta aaggaaagat tgttg 35
<210> 25
   <211> 39
   <212> DNA
   <213> Artificial
<220>
   <223> oligonucleotide as primer for PCR
<400> 25
   aattttggat cctcatgtgg tggattttcc tacatctac 39
<210> 26
   <211> 38
   <212> DNA
   <213> Artificial
<220>
   <223> oligonucleotide as primer for PCR
<400> 26
   aaaattcacg tgaacataac agaagaattt tatcaatc 38
<210> 27
   <211> 32
   <212> DNA
   <213> Artificial
<220>
   <223> oligonucleotide as primer for PCR
<400> 27
   acgcgtcgac atgtccaacc atacccatca tc 32
<210> 28
   <211> 38
   <212> DNA
   <213> Artificial
<220>
   <223> oligonucleotide as primer for PCR
<400> 28
   ttaaaatcta gattagatct gtgtagttga ttgatttg 38
<210> 29
   <211> 33
   <212> DNA
   <213> Artificial
<220>
   <223> oligonucleotide as primer for PCR
<400> 29
   ttttaaggat ccgctaaagc caagcccaca tcc 33
<210> 30
   <211> 33
   <212> DNA
   <213> Artificial
<220>
   <223> oligonucleotide as primer for PCR
<400> 30
   ttaaaatcta gattagatct gtgtagttga ttg 33
<210> 31
   <211> 36
   <212> DNA
   <213> Artificial
<220>
   <223> oligonucleotide as primer for PCR
<400> 31
   cataccgtcg acatgaagaa actagagaaa gccctg 36
<210> 32
   <211> 40
   <212> DNA
   <213> Artificial
<220>
   <223> oligonucleotide as primer for PCR
<400> 32
   cataccggat cctcaggctg catatgcccc aaaccatgtc 40
<210> 33
   <211> 39
   <212> DNA
   <213> Artificial
<220>
   <223> oligonucleotide as primer for PCR
<400> 33
   catgacgcgg ccgctatgaa gaaactagag aaagccctg 39
<210> 34
   <211> 40
   <212> DNA
   <213> Artificial
<220>
   <223> oligonucleotide as primer for PCR
<400> 34
   catgacagat ctttaggctg catatgcccc aaaccatgtc 40
<210> 35
   <211> 33
   <212> DNA
   <213> Artificial
<220>
   <223> oligonucleotide as primer for PCR
<400> 35
   catgacgtcg acatgacgac tactgcattc ctg 33
<210> 36
   <211> 36
   <212> DNA
   <213> Artificial
<220>
   <223> oligonucleotide as primer for PCR
<400> 36
   catgacagat cttcaacgtc ccgaggtcat ttgttc 36
<210> 37
   <211> 36
   <212> DNA
   <213> Artificial
<220>
   <223> oligonucleotide as primer for PCR
<400> 37
   catgacgcgg ccgctatgac gactactgca ttcctg 36
<210> 38
   <211> 35
   <212> DNA
   <213> Artificial
<220>
   <223> oligonucleotide as primer for PCR
<400> 38
   catgacagat ctcaagcgaa gtaatcccgg tggtg 35
<210> 39
   <211> 36
   <212> DNA
   <213> Artificial
<220>
   <223> oligonucleotide as primer for PCR
<400> 39
   actgtatcta gaatgaccac cacagctttc ctaatc 36
<210> 40
   <211> 39
   <212> DNA
   <213> Artificial
<220>
   <223> oligonucleotide as primer for PCR
<400> 40
   actgtaagat ctttaagtat tcactccagc acccatagc 39
<210> 41
   <211> 41
   <212> DNA
   <213> Artificial
<220>
   <223> oligonucleotide as primer for PCR
<400> 41
   catgacgcgg ccgccctatg accaccacag ctttcctaat c 41
<210> 42
   <211> 36
   <212> DNA
   <213> Artificial
<220>
   <223> oligonucleotide as primer for PCR
<400> 42
   catgacagat ctttatatga actctgagaa gtagtc 36
<210> 43
   <211> 39
   <212> DNA
   <213> Artificial
<220>
   <223> oligonucleotide as primer for PCR
<400> 43
   cataccgtcg acatgagaaa gaaattggag aaggcactg 39
<210> 44
   <211> 39
   <212> DNA
   <213> Artificial
<220>
   <223> oligonucleotide as primer for PCR
<400> 44
   cataccggat cctcatgctg catgagcacc aaaccatgc 39
<210> 45
   <211> 42
   <212> DNA
   <213> Artificial
<220>
   <223> oligonucleotide as primer for PCR
<400> 45
   catgacgcgg ccgctatgag aaagaaattg gagaaggcac tg 42
<210> 46
   <211> 39
   <212> DNA
   <213> Artificial
<220>
   <223> oligonucleotide as primer for PCR
<400> 46
   cataccagat cttcatgctg catgagcacc aaaccatgc 39
<210> 47
   <211> 39
   <212> DNA
   <213> Artificial
<220>
   <223> oligonucleotide as primer for PCR
<400> 47
   catatcgtcg acatggaata ttggaaacac acaaacagc 39
<210> 48
   <211> 38
   <212> DNA
   <213> Artificial
<220>
   <223> oligonucleotide as primer for PCR
<400> 48
   catgacgata tctagctgca gtttttcgga acttctgt 38
<210> 49
   <211> 33
   <212> DNA
   <213> Artificial
<220>
   <223> oligonucleotide as primer for PCR
<400> 49
   catactgcgg ccgctttaat tcaagagaac aat 33
<210> 50
   <211> 35
   <212> DNA
   <213> Artificial
<220>
   <223> oligonucleotide as primer for PCR
<400> 50
   catatcgata tctagctgca gtttttcgga acttc 35
<210> 51
   <211> 36
   <212> DNA
   <213> Artificial
<220>
   <223> oligonucleotide as primer for PCR
<400> 51
   catatcgtcg acatgatcat cacaaacaca atcata 36
<210> 52
   <211> 36
   <212> DNA
   <213> Artificial
<220>
   <223> oligonucleotide as primer for PCR
<400> 52
   catgaccaga tcttattgtc tatttgtcag tatata 36
<210> 53
   <211> 42
   <212> DNA
   <213> Artificial
<220>
   <223> oligonucleotide as primer for PCR
<400> 53
   catactgcgg ccgctcaaat agacataaca aaactgcaac gt 42
<210> 54
   <211> 41
   <212> DNA
   <213> Artificial
<220>
   <223> oligonucleotide as primer for PCR
<400> 54
   catatcgata tctatgcact agattgatac caacttccaa c 41
<210> 55
   <211> 36
   <212> DNA
   <213> Artificial
<220>
   <223> oligonucleotide as primer for PCR
<400> 55
   catatcgtcg acatgtggct gcagaacctg cttctc 36
<210> 56
   <211> 34
   <212> DNA
   <213> Artificial
<220>
   <223> oligonucleotide as primer for PCR
<400> 56
   catgaccaga tcttcacttc tgggctggtt ccca 34
<210> 57
   <211> 39
   <212> DNA
   <213> Artificial
<220>
   <223> oligonucleotide as primer for PCR
<400> 57
   aattttggat cctcagattc cacgattttt attagaagc 39
<210> 58
   <211> 34
   <212> DNA
   <213> Artificial
<220>
   <223> oligonucleotide as primer for PCR
<400> 58
   aaattttgtc gacatggctc ttagcaaggt caaa 34
<210> 59
   <211> 35
   <212> DNA
   <213> Artificial
<220>
   <223> oligonucleotide as primer for PCR
<400> 59
   ttaaggatcc tcacagttcc acatcattgt ctttg 35

## Description

### FIELD

The present description relates to methods of vaccination or immunization of bovines, advantageously such methods involving a prime-boost regimen, as well as vaccines or immunological or immunogenic compositions, such as DNA vaccines or immunogenic or immunological compositions, which can be used such methods.

### BACKGROUND

Deoxyribonucleic acid (DNA) molecules have been used for vaccination (Wolf et al. Science 1990. 247.1465-1468). This type of vaccination induces cellular and humoral immunity after *in vivo* transfection of cells of the subject to be vaccinated with DNA or RNA molecules encoding immunologically active proteins.

A DNA vaccine or immunogenic or immunological composition is composed of at least one plasmid which may be expressed by the cellular machinery of the subject to be vaccinated or inoculated and of a pharmaceutically acceptable vehicle or excipient. The nucleotide sequence of this plasmid encodes, *inter alia,* one or more immunogens, such as proteins or glycoproteins capable of inducing, in the subject to be vaccinated or inoculated, a cellular immune response (mobilization of the T lymphocytes) and a humoral immune response (stimulation of the production of antibodies specifically directed against the immunogen) (Davis H.L. Current Opinion Biotech. 1997. 8. 635-640).

An immunogen or immunogens derived from a pathogen may not be sufficiently effective for inducing an optimum or protective immune response in the animal to be vaccinated or inoculated. Therefore it may sometimes be useful to improve the immune response.

Various routes of administration for the DNA vaccines have been proposed (intraperitoneal, intravenous, intramuscular, subcutaneous, intradermal, mucosal, and the like). Various means of administration have also been proposed, for instance gold particles coated with DNA and projected so as to penetrate into the cells of the skin of the subject to be vaccinated (Tang et al. Nature 1992. 356. 152-154) and liquid jet injectors which make it possible to transfect both skin cells and cells of underlying tissues (Furth et al. Analytical Bioch. 1992. 205. 365-368).

Chemical compounds have been used for the *in vitro* transfection of DNA:
A/ - cationic lipids.
B/ - the polymers, such as for example SuperFect^{™} (molecules of activated dendrimers, produced by Qiagen; Xu et al. Mol. Genet. Metab. 1998. 64. 193-197), and
C/ - the biochemical agents, such as for example toxins, e.g., cholera toxins. The cationic lipids may be divided into four subgroups.
   1) The cationic lipids containing quaternary ammonium salts, such as, for example DOTMA (dioleoyloxypropyltrimethylammonium, produced by Gibco under the name Lipofectine), DOTAP (trimethyl-2,3-(octadec-9-eneoyloxy)-1-propaneammonium; Gregoriadis et al. FEBS Letters 1997. 402. 107-110), DMRIE (N-(2-hydroxyethyl)-N,N-dimethyl-2,3-bis(tetradecyloxy)-1-propaneammonium; WO-A-9634109), DLRIE (N-(2-hydroxyethyl)-N,N-dimethyl-2,3-bis(dodecyloxy)-1-propaneammonium; Felgner et al. Ann. N Y Acad. Sci. 1995. 772. 126-139).

These cationic lipids containing quaternary ammonium salts may be combined or otherwise with an additional neutral lipid, such as DOPC (dioleoylphosphatidylcholine) or DOPE (dioleoylphosphatidylethanolamine) (J.P. Behr, Bioconjugate Chemistry 1994. 5. 382-389).
2) The lipoamines, such as for example DOGS (dioctadecylamidoglycylspermine, produced by Promega under the name Transfectam; Abdallah et al. Biol. Cell. 1995. 85. 1-7), DC-Chol (dimethylaminoethane-carbamoyl-cholesterol; Gao and Huang, Biochem. Biophys. Res. Commun. 1991.179. 280-285), BGSC (bis-guanidine-spermidine-cholesterol), BGTC (bis-guanidine-trencholesterol) (Vigneron et al. Proc. Natl. Acad. Sci. USA 1996. 93. 9682-9686).
3) The cationic lipids containing quaternary ammonium salts and lipoamines, such as for example DOSPA (N,N-dimethyl-N-(2-(sperminecarboxamido)ethyl)-2,3-bis(dioleoyloxy)-1-propaneimidium pentahydrochloride, marketed by Gibco under the name LipofectAmine®; Hawley-Nelson et al. Focus 1993. 15. 73-79), GAP-DLRIE (N-(3-aminopropyl)-N,N-dimethyl-2,3-bis(dodecyloxy)-1-propaneammonium; Wheeler et al. Proc. Natl. Acad. Sci. USA 1996. 93. 11454-11459; Norman et al. Vaccine 1997.15. 801-803). And,
4) The lipids containing amidine salts, such as for example ADPDE, ADODE (Ruysschaert et al. Biochem. Biophys. Res. Commun. 1994. 203. 1622-1628).

Some of these compounds have been used in the formulation of DNA vaccines with more than mitigated results. Knowledge in the field of *in vitro* transfection is not transposable to DNA vaccination, where the objective is to ensure an optimal, and advantageously protective, immune response. Negative effects on the induction of an effective immune response, e.g., protective immune response, have even been observed with compounds known to promote transfection *in vitro.* And, some chemical compounds are toxic at high doses to the transfected cells.

In the work by Etchart (Etchart et al. J. Gen. Virol. 1997. 78. 1577-1580), the use of DOTAP did not have an adjuvant effect during the administration of the DNA vaccine by the intranasal route, whereas it had an adjuvant effect by the oral route. DOTAP has also been used in DNA vaccines encoding the influenza virus hemagglutinin (HA) on the mouse model which were administered by the intranasal route (Ban et al. Vaccine 1997. 15. 811-813), but the addition of DOTAP inhibited the immune response. The use of DC-Chol or DOTAP/DOPE in DNA vaccines encoding the hepatitis B virus surface protein (S) in mice which were administered by the intramuscular route made it possible to increase the antibody response, whereas the use of Lipofectine (or DOTMA) did not increase this response (Gregoriadis et al. FEBS Letters 1997. 402. 107-110). DC-Chol/DOPE has also been used in DNA vaccines against the human immunodeficiency virus (HIV, Env protein) in mice, with administration by the intramuscular route inducing a more effective immune response, whereas administration by the subcutaneous or intradermal route did not increase it (Ishii et al. AIDS Res. Hum. Retro. 1997. 13. 1421-1428). Clearly, many factors, including the route of administration, play into whether a compound is effective in increasing the immune respone.

The addition of certain cytokines, such as interleukins or interferons, can make it possible to enhance the immune response induced by DNA vaccines. Each cytokine triggers a reaction which is cytokine-specific and orients the immune response to a greater or lesser degree towards a cellular response or towards a humoral response (Pasquini et al. Immunol. Cell. Biol. 1997. 75. 397-401; Kim et al. J. Interferon Cytokine Res. 1999. 19. 77-84). The adjuvant effects of a cytokine obtained from a given species are not necessarily the same if the immune context varies; for instance, if a cytokine of one species is administered to another species, e.g., in a heterologous immune system. The addition of cytokine may also have no adjuvant effect, or may even result in a reversal of the effect sought, that is to say a reduction or an inhibition of the immune response. Thus, a DNA vaccine encoding a single chain of an immunoglobulin fused with GM-CSF does not increase the immune response, whereas direct administration of this fusion protein to mice is effective, in the same way as is the administration of a fusion protein consisting of Fv and of the cytokine IL-1beta or the administration of a DNA vaccine encoding the latter fusion protein (Hakim et al. J. Immunol. 1996. 157. 5503-5511). The use of plasmids co-expressing the cytokine IL-2 and the hepatitis B virus envelope protein in a fused or nonfused conformation results in an increase in the humoral and cellular immune responses (Chow et al. J. Virol. 1997. 71. 169-78). However, the use of a bicistronic plasmid encoding the human acquired immunodeficiency virus (HIV-1) glycoprotein gp120 and the cytokine IL-2 induced a lower specific anti-gp120 immune response than that obtained by the use of a monocistronic plasmid encoding only gyp120 (Barouch et al. J. Immunol 1998. 161. 1875-1882). The co-injection, into mice, of two expression vectors, one coding for the rabies virus G glycoprotein, the other for murine GM-CSF stimulates the activity of the B and T lymphocytes, whereas the co-injection with a plasmid encoding gamma-interferon (in place ofmurine GM-CSF) results in a decrease in the immune response (Xiang et al. Immunity 1995. 2. 129-135). Thus, whether a cytokine inhances an immune response depends on various factors.

Certain modifications in the antigens, such as deletions of part of the nucleotide sequence encoding the antigen, insertions of a DNA fragment into the nucleotide sequence encoding the antigen or into non-translated regions upstream or downstream, can also enhance the efficacy of DNA vaccines, for instance by enhancing the level of expression of the antigen or its presentation.

However, in practice, manipulations on the nucleotide sequences encoding the antigen may bring about a reduction or loss of the initial immunological activity. Thus, the deletion of the transmembrane domain from the gene encoding the rabies virus G antigen reduced the level of protection induced in the mouse model after administration by the intramuscular route of a DNA vaccine encoding this modified antigen (Xiang et al. Virol. 1995. 209. 569). The deletion of the transmembrane domain from the gene encoding the bovine herpesvirus (BHV) gD glycoprotein did not make it possible to increase the antibody response and induced only a partial protection in bovines vaccinated by the intramuscular route (van Drunen Little-van den Hurk et al. J. Gen. Virol. 1998. 79. 831-839). The humoral and cellular immune responses and the protection conferred are identical in guinea pigs challenged after having been immunized with the aid of either a DNA vaccine encoding the Ebola virus GP glycoprotein, or of a DNA vaccine encoding this GP glycoprotein but in a secreted form (Xu et al. Nature Medicine 1998. 4. 37-42).

The insertion of the signal sequence of the human tissue plasminogen activator (tPA) into the gene encoding the malaria Pf332 antigen did not make it possible to increase the antibody response in mice vaccinated by the intramuscular route (Haddad et al. FEMS 1997. 18. 193-202). The addition, in phase, of a tPA sequence to the gene encoding the murine rotavirus VP7 antigen also did not make it possible to increase the antibody response in mice vaccinated by the intradermal route, whereas the fusion protein consisting of the VP4 antigen and tPA allowed this increase, but without inducing an effective protection (Choi et al. Virology 1998. 250. 230-240).

Accordingly, whether a modification to a nucleotide sequence will be useful depends on many factors, and modifications carried out on the nucleotide sequence of one antigen cannot in general be directly transposed to another antigen, because antigens do not always have the same structural arrangements.

Moreover, it would be desirable to enhance or improve vaccination or immunization methods, for instance, the vaccination or immunization of bovines; and, it would be desirable to provide vaccination or immunization methods methods involving a prime-boost regimen, as well as vaccines or immunological or immunogenic compositions, such as DNA vaccines or immunogenic or immunological compositions, which can be used such methods.

### OBJECTS AND SUMMARY OF THE INVENTION

The present invention provides a kit for performing a prime-boost vaccination method against a bovine pathogen comprising:
(a) a first, priming vaccine, wherein the first, priming vaccine comprises a DNA vaccine comprising nucleic acid molecule(s) encoding and expressing *in vivo* in a bovine at least one immunogen from the bovine pathogen, wherein the immunogen is BRSV F or BRSV N or an epitope thereof or combinations thereof, and
(b) a second, boosting vaccine wherein the second, boosting vaccine against the bovine pathogen is different to the first, priming vaccine, but contains or expresses at least one immunogen of the bovine pathogen which is the same immunogen of the bovine pathogen expressed by the first, priming vaccine, wherein the second, boosting vaccine is an inactivated pathogen wherein the pathogen is bovine respiratory syncytial virus (BRSV);
wherein (a) and (b) are in separate containers, optionally with instructions for administration or use; and
wherein the bovine pathogen is bovine respiratory syncytial virus (BRSV).

In another aspect, the present invention provides the use of a bovine pathogenic agent, wherein the bovine pathogenic agent is BRSV, for the preparation of a priming DNA vaccine comprising a plasmid containing and expressing *in vivo* in a bovine at least one immunogen from said bovine pathogenic agent wherein the immunogen is BRSV F or BRSV N or an epitope thereof or combinations thereof, and for the preparation of a second vaccine comprising said bovine pathogenic agent under an inactivated form wherein the bovine pathogenic agent is bovine respiratory syncytial virus (BRSV), wherein the priming DNA vaccine is intended to be administered to a bovine first, and the inactivated vaccine is intended to be administered after the priming DNA vaccine and to the same bovine to boost the immune response against said immunogen.

The present invention provides, in another aspect, the use of a bovine pathogenic agent, wherein the bovine pathogenic agent is BRSV, to prepare an inactivated vaccine intended to vaccinate a bovine against said pathogenic agent, wherein the bovine, has previously been immunized with a DNA vaccine expressing *in vivo* at least one immunogen from the same pathogenic agent, wherein the immunogen is BRSV F or BRSV N or an epitope thereof or combinations thereof, and the bovine has developed a specific priming DNA vaccine induced immune response.

In a further aspect, the present invention provides a prime-boost Vaccination regimen for use in the treatment and/or prevention of BRSV;
wherein a priming DNA vaccine comprises a plasmid containing and expressing *in vivo* in a bovine at least one immunogen from a bovine pathogenic agent wherein the bovine pathogenic agent is BRSV and wherein the immunogen is BRSV F or BRSV N or an epitope thereof or combinations thereof; and
wherein a booster vaccine comprises a bovine pathogenic agent under an inactivated form wherein the bovine pathogenic agent is bovine respiratory syncytial virus (BRSV);
wherein the priming DNA vaccine is intended to be administered to a bovine first, and the inactivated vaccine is intended to be administered after the priming DNA vaccine and to the same bovine to boost the immune response against said immunogen.

In another aspect, the present invention provides an inactivated vaccine for use in the treatment and/or prevention of BRSV;
wherein the inactivated vaccine comprises a bovine pathogenic agent, wherein the bovine pathogenic agent is BRSV, and wherein the pathogenic agent is inactivated;
wherein the inactivated vaccine is intended to be administered to a bovine that has previously been immunized with a DNA vaccine expressing *in vivo* at least one immunogen from the same pathogenic agent, wherein the immunogen is BRSV F or BRSV N or an epitope thereof or combinations thereof, and the bovine has developed a specific priming DNA vaccine induced immune response.

It has been found that DNA vaccination or immunization of animals which can be given DNA vaccination or immunization, e.g., mammals, avians, reptiles, advantageously bovines (e.g., cows, calves, bull, cattle, buffalo "beefalo" and the like), can be improved by a vaccination or immunization regimen; for instance, administering one or more DNA vaccines or immunological or immunogenic compositions as a "prime" and thereafter administering one or more subunit (e.g., antigen(s), immunogen(s) or epitope(s) preparation(s) - "subunit(s)" of the pathogen), and/or inactivated pathogen and/or attenuated pathogen vaccine or immunological or immunogenic compositions and/or a recombinant or modified vector, e.g., virus, bacterial or yeast, vaccine or immunogenic or immunological compositions (which contain an *in vivo* expression vector, e.g., a modified or recombinant virus, bacteria, yeast or other expression vector).

The prime-boost regimen according to the description can be used in animals of any age, advantageously young animals (e.g., animals that have detectable maternal antibodies and/or are suckling or nursing or breast-feeding, such as a young calve - a calve that has detectable maternal antibodies and/or is suckling or nursing or breast-feeding), pre-adult animals (animals that are older than being a young animal but have not yet reached maturity or adulthood or an age to mate or reproduce), adult animals (e.g, animals that are of an age to mate or reproduce or are beyond such a period in life), and it is advantageous to employ the prime-boos regimen in pregnant females or females prior to giving birth or insemination.

The prime-boost regimen is especially advantageous to practice in a young animal, e.g., a young bovine or calve, as it allows vaccinatation or immunization at an early age, for instance, the first administration in the prime-boost regimen or the prime can be administered to a young animal can be at an age at which the the young animal has maternal antibodies. Another advantage of this regimen is that it can provide a degree of safety for pregnant females, e.g., cows, present in the same location or in close proximity to the young or to each other, e.g. at the same farm or that share common grazing area.

Thus, thedescriptionprovides a prime-boost immunization or vaccination method advantageously practiced in bovines against one or more pathogens of bovines, and the method may be practiced upon a young animal, such as a young calve, for instance, wherein the priming is done at a time that the young animal has maternal antibodies against the bovine pathogen, with the boost advantageously at a time when maternal antibodies may be waning or decreasing or normally not present, such as during a period of time post-breastfeeding.

The bovine pathogen against which the prime-boost regimen can be employed includes: bovine respiratory syncitial virus (BRSV), bovine parainfluenza virus type 3 (bPI-3), bovine herpesvirus type 1 (BHV-1) (responsible for infectious bovine rhinotracheitis (IBR)), mucosal disease virus and bovine pestivirus type 1 or type 2 (bovine viral diarrhea virus or BVDV-1 and BVDV-2)). Advantageously, the prime-boost regimen of thedescriptionis practiced against BRSV.

The description further comprehends the compositions and kits including one or more DNA vaccines or immunogenic or immunological compositions which may be used in the prime-boost regimen of the description and which make it possible to obtain an improved or advantageously effective and/or protective immune protection in cattle, comprising at least one valency selected from the group consisting of the BRSV, bPI-3, BHV-1 and BVDV (comprising at least one plasmid that contains and expresses a nucleic acid molecule encoding at least one immunogen, antigen or epitope of BRSV, bPI-3, BHV-1 or BVDV).

Accordingly, the description also involves kits for performing a prime-boost regimen comprising or consisting essentially of a priming vaccine or immunological or immunogenic composition and a boost vaccine or immunological or immunogenic compositions, in separate containers, optionally with instructions for admixture and/or administration.

The description provides a prime-boost immunization or vaccination method of a bovine (e.g., cow, bull, calve) against at least one bovine pathogen comprising administering to the bovine a priming DNA vaccine or immunological or immunogenic composition comprising nucleic acid molecule(s) encoding and expressing *in vivo* an immunogen(s), antigen(s) or epitope(s) from the pathogen, and thereafter administering a boosting vaccine or immunogenic or immunological composition that presents to the bovine's immune system the same immunogen, antigen or epitope. The boosting vaccine or immunogenic or immunological composition is advantageously different than the DNA vaccine or immunogenic or immunological composition. For instance, the boosting vaccine or immunogenic or immunological composition can be an inactivated pathogen and/or an attenuated pathogen and/or a subunit (advantageously the antigen, immunogen and/or epitope expressed by the DNA vaccine or immunogenic or immunological composition) and/or a recombinant or modified vector, e.g., virus, vaccine or immunogenic or immunological composition. A recombinant or modified vector is advantageously an *in vivo* expression vector, such as a modified or recombinant bacteria, yeast, virus, e.g. poxvirus, adenovirus, herpesvirus, comprising nucleic acid molecule(s) encoding and expressing *in vivo* the immunogen(s), antigen(s) or epitope(s) from the pathogen expressed by the DNA vaccine or immunogenic or immunological composition. The boost is advantageously performed with an inactivated vaccine or immunogenic or immunological composition, or with a vaccine or immunogenic or immunological composition comprising a recombinant live viral vector, such as a recombinant poxvirus, that comprises nucleic acid molecule(s) encoding and express(es) *in vivo* the immunogen(s), antigen(s) or epitope(s) from the pathogen expressed by the DNA vaccine or immunogenic or immunological composition. Thus, it is advantageous that the boost either comprises the immunogen, antigen or epitope expressed by the DNA vaccine or immunogenic or immunological composition or expresses *in vivo* the same immunogen, antigen or epitope expressed by the DNA vaccine or immunogenic or immunological composition.

The terms "immunogenic composition" and "immunological composition" and "immunogenic or immunological composition" cover any composition that elicits an immune response against the targeted pathogen; for instance, after administration or injection into the bovine, elicits an immune response against the targeted pathogen. The terms "vaccinal composition" and "vaccine" and "vaccine composition" covers any composition that induces a protective immune response against the targeted pathogen or which efficaciously protects against the pathogen; for instance, after administration or injection into the bovine, elicits a protective immune response against the targeted pathogen or provides efficacious protection against the pathogen. Furthermore, while the text speaks of "immunogen, antigen or epitope", an immunogen can be an antigen or an epitope of an antigen.

The term of "prime-boost" refers to the successive administrations of two different types of vaccine or immunogenic or immunological compositions having at least one immunogen, antigen or epitope in common. The priming administration (priming) is the administration of a first vaccine or immunogenic or immunological composition type and may comprise one, two or more administrations. The boost administration is the administration of a second vaccine or immunogenic or immunological composition type and may comprise one, two or more administrations, and, for instance, may comprise or consist essentially of annual administrations.

Thus, the description comprehends administering to a bovine a priming composition comprising a DNA vaccines or immunogenic or immunological composition against a bovine pathogen comprising at least one plasmid that contains and expresses in a bovine host cell a nucleotide sequence encoding an immunogen, antigen or epitope of the bovine pathogen, and thereafter a boosting composition that comprises the bovine pathogen as an inactivated pathogen, or the bovine pathogen as an attenuated pathogen, or the immunogen, antigen or epitope expressed by the DNA vaccine or immunogenic or immunological composition, or a recombinant or modified vector, e.g., virus, such as a recombinant or modified herpesvirus, adenovirus or poxvirus (advantageously poxvirus, such as a vaccinia, canarypox or fowlpox virus) that contains and expresses in a bovine host cell a nucleotide sequence encoding the immunogen, antigen or epitope of the bovine pathogen expressed by the DNA vaccine or immunogenic or immunological composition. The bovine pathogen can be BRSV, bPI-3, BHV-1 or BVDV.

The DNA vaccine or immunogenic composition can contain a cationic lipid containing a quaternary ammonium salt, of the formula 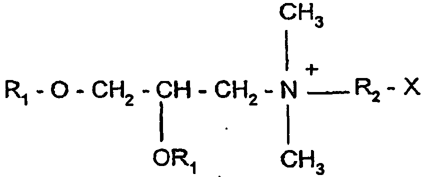 in which R₁ is a saturated or unsaturated linear aliphatic radical having 12 to 18 carbon atoms, R₂ is an aliphatic radical containing 2 or 3 carbon atoms, and X a hydroxyl or amine group. Advantageously, this compound is DMRIE (N-(2-hydroxyethyl)-N,N-dimethyl-2,3 bis(tetradecyloxy)-1-propanammonium). This compound can be combined with a neutral lipid, such as, DOPE (dioleoyl-phosphatidyl-ethanolamine). When the compound is DMRIE and the neutral lipid is DOPE, and they are combined, they form DMRIE-DOPE.

Alternatively or additionally, the DNA vaccine or immunogenic composition can contain bovine GM-CSF, or an expression vector that contains and expresses in a bovine host cell a nucleotide sequence encoding bovine GM-CSF. The expression vector that contains and expresses the bovine GM-CSF can be a plasmid, or a recombinant or modified vector such as a recombinant or modified virus, bacteria, yeast. The plasmid in the DNA vaccine or immunogenic composition that expresses the immunogen, antigen or epitope of the bovine pathogen can also express bovine GM-CSF.

Even further alternatively or additionally, in the DNA vaccine or immunogenic composition, the nucleotide sequence encoding the immunogen, antigen or epitope of the bovine pathogen can have deleted therefrom a portion encoding a transmembrane domain.

Yet even further alternatively or additionally, the plasmid in the DNA vaccine or immunogenic composition can further contain and express in a bovine host cell a nucleotide sequence encoding a heterologous tPA signal sequence such as human tPA and/or a stabilizing intron, such as intron II of the rabbit beta-globin gene.

Advantageously, the bovine pathogen is BRSV; for example, the immunogen can be BRSV F or G or N. Even more advantageously, the immunogen is BRSV F or G, modified by substitution of the BRSV F and/or G signal sequence with a human tPA signal sequence, and/or by deletion of the transmembrane domain and/or cytoplasmic tail. The coding for the F protein can also contain a deletion of nucleotides upstream from the transmembrane domain and corresponding to 1 and up to 92 amino acids.

Thus, the DNA vaccine or immunogenic composition can comprise a first plasmid that contains and expresses in a bovine host cell a nucleotide sequence encoding BRSV F, modified by substitution of the BRSV F signal sequence with a human tPA signal sequence and deletion of the transmembrane domain and contiguous C-terminal portion (cytoplasmic tail) optionally also with deletion of the upstream region described above; and a second plasmid that contains and expresses in a bovine host cell a nucleotide sequence encoding BRSV N and/or BRSV G, modified by substitution of the BRSV G signal sequence with a human tPA signal sequence and deletion of the transmembrane domain and contiguous C-terminal portion (cytoplasmic tail); and wherein the cationic lipid is present, e.g., DMRIE, and the neutral lipid is also present, e.g., DOPE, for instance whereby the vaccine or immunogenic composition comprises DMRIE-DOPE. This DNA vaccine or immunogenic composition can further comprise bovine GM-CSF or an expression vector (e.g., plasmid) that contains and expresses in a bovine host cell a nucleotide sequence encoding bovine GM-CSF. Additionally or alternatively, the DNA vaccine or immunogenic composition can contain a plasmid that expresses all of these "optimised" BRSV immunogens, and advantageously further contains the cationic lipid is present, e.g., DMRIE, and the neutral lipid is present, e.g., DOPE, for instance, whereby the DNA vaccine or immunogenic composition comprises DMRIE-DOPE; and, this DNA vaccine or immunogenic composition can further comprise bovine GM-CSF or an expression vector (e.g., plasmid) that contains and expresses in a bovine host cell a nucleotide sequence encoding bovine GM-CSF.

In the practice of the description the bovine pathogen can be bovine pathogen is BHV-1, and the immunogen can be BHV-1 gB and/or BHV-1 gC and/or BHV-1 gD. The immunogen can be BHV-1 gB, modified by substitution of the BHV-1 gB signal sequence with a human tPA signal sequence, and/or by deletion of the transmembrane domain and/or BHV-1 gC, modified by substitution of the BHV-1 gC signal sequence with a human tPA signal sequence, and/or by deletion of the transmembrane domain and/or BHV-1 gD, modified by substitution of the BHV-1 gD signal sequence with a human tPA signal sequence, and/or by deletion of the transmembrane domain.

Thus, the DNA vaccine or immunogenic composition can comprise a first plasmid that contains and expresses in a bovine host cell a nucleotide sequence encoding BHV-1 gB, modified by substitution of the BHV-1 gB signal sequence with a human tPA signal sequence and deletion of the transmembrane domain and contiguous C-terminal portion; a second plasmid that contains and expresses in a bovine host cell a nucleotide sequence encoding BHV-1 gC, modified by substitution of the BHV-1 gC signal sequence with a human tPA signal sequence and deletion of the transmembrane domain and contiguous C-terminal portion; a third plasmid that contains and expresses in a bovine host cell a nucleotide sequence encoding BHV-1 gD, modified by substitution of the BHV-1 gD signal sequence with a human tPA signal sequence and deletion of the transmembrane domain and contiguous C-terminal portion; and wherein the cationic lipid is present, e.g., DMRIE, and the neutral lipid is present, e.g., DOPE, for instance, whereby the DNA vaccine or immunogenic composition comprises DMRIE-DOPE. This DNA vaccine or immunogenic composition can further comprise bovine GM-CSF or an expression vector (e.g., plasmid) that contains and expresses in a bovine host cell a nucleotide sequence encoding bovine GM-CSF. Additionally or alternatively, the DNA vaccine or immunogenic composition can contain a plasmid that expresses all of these "optimised" BHV-1 immunogens, and advantageously further contains the cationic lipid is present, e.g., DMRIE, and the neutral lipid is present, e.g., DOPE, for instance, whereby the DNA vaccine or immunogenic composition comprises DMRIE-DOPE; and, this DNA vaccine or immunogenic composition can further comprise bovine GM-CSF or an expression vector (e.g., plasmid) that contains and expresses in a bovine host cell a nucleotide sequence encoding bovine GM-CSF.

In the practice of the description the bovine pathogen can be BVDV, and the immunogen the EO protein (gp48) and/or the E2 protein (gp53) Thus, the DNA vaccine or immunogenic or immunological composition can comprise the nucleotide sequence(s) encoding BVDV EO and/or E2 proteins. The immunogen can be BVDV EO, modified by being encoded by a nucleic acid into which has been inserted coding for a signal sequence, e.g., a human tPA signal sequence, and/or into which has been inserted an intron such as intron II of rabbit beta-globin, and/or BVDV E2 modified by being encoded by a nucleic acid into which has been inserted coding for a signal sequence, e.g., a human tPA signal sequence, and/or which has had deleted therefrom coding for the transmembrane domain of E2 and/or the cytoplasmic tail and/or into which has been inserted an intron such as intron II of rabbit beta-globin.

Thus, the DNA vaccine or immunogenic composition can comprise a first plasmid that contains and expresses in a bovine host cell a nucleotide sequence encoding BVDV E0, modified by containing a human tPA signal sequence and intron II of rabbit beta-globin; and a second plasmid that contains and expresses in a bovine host cell a nucleotide sequence encoding BVDV E2, modified containing a human tPA signal sequence and intron II of rabbit beta-globin and deletion of the transmembrane domain and cytoplasmic tail; and wherein the cationic lipid is present, e.g., DMRIE, and the neutral lipid is present, e.g., DOPE, for instance, whereby the DNA vaccine or immunogenic composition comprises DMRIE-DOPE. This DNA vaccine or immunogenic composition can further comprise bovine GM-CSF or an expression vector (e.g., plasmid) that contains and expresses in a bovine host cell a nucleotide sequence encoding bovine GM-CSF. Additionally or alternatively, the DNA vaccine or immunogenic composition can contain a plasmid that expresses all of these "optimised" BVDV immunogens, and advantageously further contains the cationic lipid is present, e.g., DMRIE, and the neutral lipid is present, e.g., DOPE, for instance, whereby the DNA vaccine or immunogenic composition comprises DMRIE-DOPE; and, this DNA vaccine or immunogenic composition can further comprise bovine GM-CSF or an expression vector (e.g., plasmid) that contains and expresses in a bovine host cell a nucleotide sequence encoding bovine GM-CSF. As there are different types of BVDV, namely BVDV-1 and BVDV-2, the E0 and E2 can be from either or both of the BVDV types. Thus, BVDV DNA vaccines or immunogenic compositions of the description can contain a plasmid or plasmids containing and expressing nucleic acid molecules of BVDV-1, or BVDV-2, or both BVDV-1 and BVDV-2. Accordingly, the foregoing "two plasmid" composition can be a "four plasmid" composition to address both types of BVDV. Thus, the description comprehends a mixture of plasmids. The mixture may comprise at least two expression plasmids, each expressing a different immunogen (E0 or E2) and/or obtained from a different type of BVDV (BVDV-1 of BVDV-2), such as a mixture made of four plasmids expressing BVDV-1 E0, BVDV-1 E2, BVDV-2 E0 and BVDV-2 E2.

In the practice of the description the bovine pathogen can be bPI-3; for example, the immunogen can be bPI-3 F or HN. Even more advantageously, the immunogen is bPI-3 F or HN, modified by substitution of the bPI-3F and/or HN signal sequence with a human tPA signal sequence, and/or by deletion of the transmembrane domain and/or cytoplasmic tail, and/or by insertion into the nucleic acid molecule coding therefor of an intron, such as intron II of rabbit beta-globin.

Thus, the DNA vaccine or immunogenic composition can comprise a first plasmid that contains and expresses in a bovine host cell a nucleotide sequence encoding bPI-3 F, modified by insertion of intron II of rabbit beta-globin, substitution of the bPI-3 F signal sequence with a human tPA signal sequence and deletion of the transmembrane domain and cytoplasmic tail; and a second plasmid that contains and expresses in a bovine host cell a nucleotide sequence encoding bPI-3 HN, modified by insertion of intron II of rabbit beta-globin, substitution of the bPI-3 HN signal sequence with a human tPA signal sequence and deletion of the transmembrane domain and cytoplasmic tail; and wherein the cationic lipid is present, e.g., DMRIE, and the neutral lipid is also present, e.g., DOPE, for instance whereby the vaccine or immunogenic composition comprises DMRIE-DOPE. This DNA vaccine or immunogenic composition can further comprise bovine GM-CSF or an expression vector (e.g., plasmid) that contains and expresses in a bovine host cell a nucleotide sequence encoding bovine GM-CSF. Additionally or alternatively, the DNA vaccine or immunogenic composition can contain a plasmid that expresses all of these "optimised" bPI-3 immunogens, and advantageously further contains the cationic lipid is present, e.g., DMRIE, and the neutral lipid is present, e.g., DOPE, for instance, whereby the DNA vaccine or immunogenic composition comprises DMRIE-DOPE; and, this DNA vaccine or immunogenic composition can further comprise bovine GM-CSF or an expression vector (e.g., plasmid) that contains and expresses in a bovine host cell a nucleotide sequence encoding bovine GM-CSF.

And, in general, DNA plasmids for DNA vaccines or immunogenic or immunological compositions, and DNA vaccines or immunogenic or immunological compositions employed in the "priming" of the herein prime-boost method may be as in U.S. Patent No. 6,376,473 and U.S. applications Serial Nos. 10/085,519, 09/766,442, 09/760,574, 60/193,126, 09/232,468, 09/232,469, and 09/232,279, and French application No. 00 00798, filed January 21, 2000.

These and other embodiments are disclosed or are obvious from and encompassed by, the following Detailed Description.

### BRIEF DESCRIPTION OF DRAWINGS

The following Detailed Description, given by way of example, and not intended to limit the invention to specific embodiments described, may be understood in conjunction with the accompanying Figures, incorporated herein by reference, in which:
Figure 1 shows plasmid pVR1012;
Figure 2 shows plasmid pAB 110;
Figure 3 shows a graph representing the evolution of the rectal temperature after challenge according to example 11;
Figure 4 shows a graph representing the respiratory rate after challenge according to example 11;
Figure 5 shows a graph representing the clinical scores after challenge according to example 11;
Figure 6 shows a graph representing the lung lesion scores after challenge according to example 11;
Figure 7 shows a graph representing the viral excretion after challenge according to example 11; and,
Figure 8 shows a graph representing the memory BRSV-specific IFN□+ T cell response after challenge according to example 11.

### Sequence listing:

SEQ ID NO 1: oligonucleotide PB326
SEQ ID NO 2: oligonucleotide PB329
SEQ ID NO 3: oligonucleotide SB090
SEQ ID NO 4: oligonucleotide SB091
SEQ ID NO 5: oligonucleotide LF001
SEQ ID NO 6: oligonucleotide LF002
SEQ ID NO 7: oligonucleotide PB234
SEQ ID NO 8: oligonucleotide PB235
SEQ ID NO 9: oligonucleotide PB511
SEQ ID NO 10: oligonucleotide PB512
SEQ ID NO 11: oligonucleotide SB221
SEQ ID NO 12: oligonucleotide SB222
SEQ ID NO 13: oligonucleotide PB507
SEQ ID NO 14: oligonucleotide PB508
SEQ ID NO 15: oligonucleotide PB513
SEQ ID NO 16: oligonucleotide PB514
SEQ ID NO 17: oligonucleotide SB223
SEQ ID NO 18: oligonucleotide SB224
SEQ ID NO 19: oligonucleotide PB497
SEQ ID NO 20: oligonucleotide PB498
SEQ ID NO 21: oligonucleotide SB225
SEQ ID NO 22: oligonucleotide SB226
SEQ ID NO 23: oligonucleotide SB210
SEQ ID NO 24: oligonucleotide SB211
SEQ ID NO 25: oligonucleotide SB212
SEQ ID NO 26: oligonucleotide SB220
SEQ ID NO 27: oligonucleotide SB213
SEQ ID NO 28: oligonucleotide SB214
SEQ ID NO 29: oligonucleotide SB215
SEQ ID NO 30: oligonucleotide SB216
SEQ ID NO 31: oligonucleotide LF050
SEQ ID NO 32: oligonucleotide LF051
SEQ ID NO 33: oligonucleotide LF052
SEQ ID NO 34: oligonucleotide LF053
SEQ ID NO 35: oligonucleotide LF039
SEQ ID NO 36: oligonucleotide LF040
SEQ ID NO 37: oligonucleotide LF041
SEQ ID NO 38: oligonucleotide LF042
SEQ ID NO 39: oligonucleotide LF043
SEQ ID NO 40: oligonucleotide LF044
SEQ ID NO 41: oligonucleotide LF045
SEQ ID NO 42: oligonucleotide LF046
SEQ ID NO 43: oligonucleotide LF064
SEQ ID NO 44: oligonucleotide LF065
SEQ ID NO 45: oligonucleotide LF066
SEQ ID NO 46: oligonucleotide LF067
SEQ ID NO 47: oligonucleotide LF047
SEQ ID NO 48: oligonucleotide LF048
SEQ ID NO 49: oligonucleotide LF058
SEQ ID NO 50: oligonucleotide LF059
SEQ ID NO 51: oligonucleotide LF060
SEQ ID NO 52: oligonucleotide LF061
SEQ ID NO 53: oligonucleotide LF062
SEQ ID NO 54: oligonucleotide LF063
SEQ ID NO 55: oligonucleotide LF054
SEQ ID NO 56: oligonucleotide LF055
SEQ ID NO 57: oligonucleotide FC129
SEQ ID NO 58: oligonucleotide FC130
SEQ ID NO 59: oligonucleotide FC131

### DETAILED DESCRIPTION

As discussed herein, thedescriptioninvolves a prime-boost method that is advantageously practiced in bovines, such as young calves that can have maternal antibodies against the pathogenic agent against which immunization or vaccination is directed.

The DNA vaccine or immunological or immunogenic composition can be administered to the young animal, calve; wherein a "young animal" or "young calve" is a calve from calving up to and including 12 weeks of age, such as from calving up to and including 6 weeks of age, advantageously from calving up to and including 4 weeks of age, e.g., from calving up to and including 3 weeks of age.

The boost administration advantageously may be administered from about 2 weeks to about 5 months after the priming administration, such as from about 3 to 6 weeks after the priming administration, and advantageously about 4 weeks after the priming administration the priming administration. A second administration of the boost vaccine or immunological or immunogenic composition may occur, such as when calves are transferred to finishing units.

The DNA vaccine or immunogenic or immunological composition comprises, as an active ingredient, a plasmid comprising a nucleic acid molecule which codes for an immunogen, antigen or epitope of a pathogenic agent, advantageously a bovine pathogenic agent. The term plasmid covers a DNA transcription unit comprising a polynucleotide sequence comprising the sequence of the nucleic acid molecule to be expressed and the elements necessary for its expression in vivo. The circular plasmid form, supercoiled or otherwise, is within the scope of the description. The linear form also falls within the scope of this description.

Each plasmid comprises a promoter, for expression of the nucleic acid molecule encoding the immunogen, antigen or epitope, and thus, the nucleic acid molecule encoding the immunogen, antigen or epitope is operably linked to or under the control of the promoter. Advantageously, the promoter is a eukaryotic promoter, even more advantageously a strong promoter, such as a strong eukaryotic promoter, e.g., a cytomegalovirus early promoter CMV-IE, of human or murine origin, or optionally of another origin such as murine, rat or guinea pig. Functional subfragments of these promoters, i.e., portions of these promoters that maintain an adequate promoting activity, are included within the present description e.g. truncated CMV-IE promoters according to WO98/00166 or U.S. Patent No. 6,156,567 can be used. A promoter in the practice of the description consequently includes derivatives and subfragments of a full-length promoter that maintain an adequate promoting activity and hence function as a promoter, advantageously promoting activity substantially similar to that of the actual or full-length promoter from which the derivative or subfragment is derived, e.g., akin to the activity of the truncated CMV-IE promoters of U.S. Patent No. 6,156,567 to the activity of full-length CMV-IE promoters. Thus, a CMV-IE promoter in the practice of the description can comprise or consist essentially of or consist of the promoter portion of the full-length promoter and/or the enhancer portion of the full-length promoter, as well as derivatives and subfragments.

More generally, the promoter can be of viral origin or of cellular origin. As a viral promoter other than CMV-IE, the promoter can be the SV40 virus early or late promoter or the Rous Sarcoma virus LTR promoter. The promoter can also be a promoter from a bovine pathogenic agent, e.g., from the pathogenic agent from which the nucleic acid molecule encoding the antigen, immunogen or epitope is derived, for example the promoter specific to the nucleic acid molecule encoding the antigen, immunogen or epitope. A cellular promoter that can be employed in the practice of the description is a promoter of a cytoskeleton gene, such as, for example, the desmin promoter, or alternatively the actin promoter. When several genes are present in the same plasmid, they may be provided in the same transcription unit or in several different units.

The plasmid(s) in the DNA vaccines or immunogenic or immunological compositions are in a veterinarily acceptable vehicle or excipient. In general, the vehicle or excipient (Remington's Pharmaceutical Sciences, by E. W. Martin, Mack Publishing Co., Easton, PA, 15th Edition, 1975) can be any usual injectable fluid such as water, physiological saline, balanced salt solution, aqueous dextrose, glycerol or the like. Hence, the DNA vaccines or immunogenic or immunological compositions can advantageously contain a veterinarily acceptable vehicle or excipient.

The DNA vaccines or immunogenic or immunological compositions according to the description can also be adjuvanted, i.e., they can contain an adjuvant. Examples of adjuvants include non-methylated CpG groups (Klinman D. M. et al., Proc. Natl. Acad. Sci. USA 93:2879-2883, 1996; WO 98/16247), or aluminum hydroxide, aluminum phosphate, aluminum oxide ("Vaccine Design, The subunit and adjuvant approach," Pharmaceutical Biotechnology, vol. 6, Edited by Micheal F. Powell and Mark J. Newman, 1995, Plenum Press New York). Adjuvants advantageously employed in the practice of the invention are the cationic lipids, such as those containing a quaternary ammonium salt of formula: 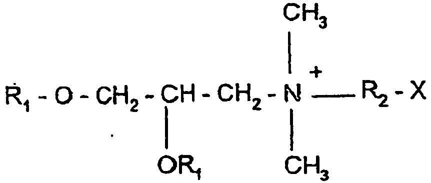 in which R₁ is a saturated or unsaturated linear aliphatic radical having 12 to 18 carbon atoms, R₂ is another aliphatic radical containing 2 or 3 carbon atoms, and X a hydroxyl or amine group.

Advantageously, this cationic lipid is DMRIE (N-(2-hydroxyethyl)-N,N-dimethyl-2,3-bis(tetradecyloxy)-1-propanammonium; WO-A-9634109).

The cationic lipid may be combined with a neutral lipid, such as, DOPE (dioleoyl-phosphatidyl-ethanolamine). DMRIE and DOPE form DMRIE-DOPE.

Advantageously, the plasmid is mixed with the cationic lipid immediately before use and it is even more advantageous, before its administration to the animal, to allow the mixture thus prepared to form a complex, for example for sitting after admixture for a period ranging from about 10 to about 60 minutes, such as about 30 minutes.

When the neutral lipid, e.g., DOPE, is present, the cationic lipid:neutral lipid, e.g., DMRIE:DOPE, molar ratio advantageously ranges from about 95:5 to about 5:95, and is even more advantageously about 1:1.

The plasmid:cationic lipid, e.g., DMRIE, or cationic lipid-neutral lipid, e.g., DMRIE-DOPE, adjuvant weight ratio may range from about 50:1 to about 1:10, in such as from about 10:1 to about 1:5, advantageously from about 1:1 to about 1:2.

The DNA vaccines or immunogenic or immunological compositions according to the description can be formulated with a liposome, in the presence or not of an adjuvant as described above.

Additionally or alternatively, DNA vaccines or immunogenic or immunological compositions of the description contain GM-CSF (granulocyte macrophage-colony stimulating factor, Clark S.C. et al. Science 1987. 230.1229; Grant S.M. et al. Drugs 1992. 53. 516), or an expression vector that so expresses GM-CSF, with the "expression vector" including the plasmid that expresses the antigen, immunogen or epitope of the bovine pathogen. Thus, to the DNA vaccine or immunogenic or immunological composition is added GM-CSF or a vector that expresses GM-CSF, e.g. added to the non-adjuvanted or adjuvanted and/or liposome formulated vaccines or immunogenic or immunological compositions; or, the DNA plasmid that expresses the antigen, immunogen or epitope of the bovine pathogen is constructed so that it also expresses GM-CSF. If an expression vector is providing the GM-CSF, a nucleic acid sequence encoding GM-CSF is in the expression vector under conditions allowing its expression *in vivo* (e.g., it is operably linked to s suitable promoter). Advantageously, the expression vector that expresses the GM-CSF is a plasmid, e.g. the plasmid containing the nucleotide sequence encoding the immunogen(s) of interest (encoding the bovine antigen, immunogen or epitope), or another plasmid. The GM-CSF is advantageously bovine GM-CSF (Maliszewski et al., Molec. Immunol., 1988, 25, 843-850).

In the vaccines or immunogenic or immunological compositions according to the description e.g. in the non-adjuvanted or adjuvanted and/or liposome formulated vaccines or immunogenic or immunological compositions, containing or not GM-CSF or an expression vector expressing GM-CSF, the nucleotide sequence(s) encoding the immunogen are in an optimised or modified form. Optimization is understood to mean any modification of the nucleotide sequence which manifests itself at least by a higher level of expression of this nucleotide sequence, and/or by an increase in the stability of the messenger RNA encoding this antigen, and/or by the triggered secretion of this antigen into the extracellular medium, and which may have as direct or indirect consequence an increase in the immune response induced.

In the present description the optimization of the nucleotide sequence(s) encoding the immunogen may consist in the deletion of the fragment of the nucleotide sequence encoding the transmembrane domain of the immunogen (with deletion understood to mean the complete deletion or a partial deletion or disruption sufficient for the transmembrane domain to no longer, or no longer substantially, be functional, such that deletion of the nucleotide sequence encoding the transmembrane domain can include disruption of the coding sequence), and/or the addition, in frame, of a nucleotide sequence encoding a heterologous (to the pathogen) tPA signal sequence such as the human tPA signal sequence (Montgomery et al. Cell. Mol. Biol. 1997. 43. 285-292; Harris et al. Mol. Biol. Med 1986. 3. 279-292), and/or in insertion of a stabilizing intron, advantageously upstream of the nucleic acid molecule (encoding the immunogen, antigen or epitope) to be expressed, such as intron II or rabbit beta-globin. The deletion of the DNA fragment encoding the transmembrane domain of the antigen of interest promotes the secretion, into the extracellular medium, of the antigens thus truncated and thus increases the likelihood of the antigens coming into contact with the cells of the immune system. The insertion of the nucleotide sequence encoding the tPA signal facilitates the translatability of the messenger RNA to which the tPA signal is joined, and thus increases the level of expression of this messenger RNA and therefore the production of antigens. The tPA signal also plays a role in the secretion of the antigen synthesized. Other nucleotide sequences encoding signal peptides may be used, such as those for the signal peptide of melittin obtained from bees (Sisk W.P. et al., 1994, J. Virol., 68, 766-775). The insertion of a stabilizing intron into the nucleic acid molecule encoding the antigen of interest avoids the aberrant splicings of its messenger RNA and maintains the physical integrity of the latter.

Advantageously, the tPA signal is of human origin. The nucleotide sequence of the human tPA signal is accessible from the GenBank database under the accession number NM_000930. Advantageously, the intron is intron II of the rabbit beta-globin gene (van Ooyen et al. Science 1979. 206. 337-344), whose nucleotide sequence is accessible from the GenBank database under the accession number V00882 and designated by a reference under intron No. 2.

In an embodiment, the present invention involves vaccination or immunization against bovine respiratory syncytial virus (BRSV).

BRSV virus is a Paramyxovirus, also a member of the *Paramyxoviridae* family (Baker et al., Vet. Clin. North Am. Food Anim. Pract., 1997, 13, 425-454).

Nucleotide sequences encoding the F glycoprotein, the N protein and the G glycoprotein are known and accessible from the GenBank database respectively under the accession number Y17970, M35076 and U33539. The DNA vaccine or immunogenic or immunological composition used in the prime-boost regimen against BRSV may thus comprise the nucleotide sequence(s) encoding the F, N and/or G proteins.

The nucleotide sequences and the antigens encoded therefrom may be modified. This can be carried out by substitution, by a "signal" sequence, such as the signal sequence of human tPA, for the signal sequence of the F protein of BRSV and/or for the G envelope glycoprotein of BRSV, and/or by the deletion of the DNA fragment encoding the transmembrane domain of F and/or of G. The deletion of the DNA fragment encoding the transmembrane domain of one of these proteins is advantageously accompanied by the deletion of the cytoplasmic tail. It is possible to increase the level of expression of the F glycoprotein by further deleting the nucleotide sequence upstream from the transmembrane domain and corresponding to 1 and up to 92 amino acids.

Advantageously, the DNA vaccine or immunogenic or immunological composition against BRSV comprises the nucleotide sequence encoding the F protein, or the nucleotide sequence encoding the N protein, or the nucleotide sequences encoding the F protein and the N protein.

In a particular embodiment, the DNA vaccine or immunogenic or immunological composition comprises the nucleotide sequences encoding the F protein, wherein the nucleotide sequence encoding the F protein is modified. This modification is chosen from among:
i. substitution, of the "signal" sequence with a heterologous signal sequence, such as that of the tPA of human origin,
ii. deletion of the DNA fragment encoding the transmembrane domain of F and advantageously of the cytoplasmic tail,
iii. deletion of the DNA fragment encoding the transmembrane domain, of the cytoplasmic tail and of the upstream region described above,
iv. substitution, of the "signal" sequence with a heterologous signal sequence, such as that of the tPA of human origin, and deletion of the DNA fragment encoding the transmembrane domain of F and advantageoulsy of the cytoplasmic tail, or
v. substitution, of the "signal" sequence with a heterologous signal sequence, such as that of the tPA of human origin, and deletion of the DNA fragment encoding the transmembrane domain of F, of the cytoplasmic tail and of the upstream region described above.

In a second embodiment, the DNA vaccine or immunogenic or immunological composition comprises the nucleotide sequences encoding the F protein and the N protein, either one plasmid containing both nucleotide sequences, or two separate plasmids, one containing the nucleotide sequence encoding the F protein, and one containing the nucleotide sequence encoding the N protein.

In a third embodiment, the DNA vaccine or immunogenic or immunological composition comprises the nucleotide sequences encoding F protein and the N protein, either one plasmid containing both nucleotide sequences, or two separate plasmids, one containing the nucleotide sequence encoding the F protein, and one containing the nucleotide sequence encoding the N protein, wherein the nucleotide sequence encoding the F protein is modified as described above, with the modification being chosen among those described under paragraphs i to v.

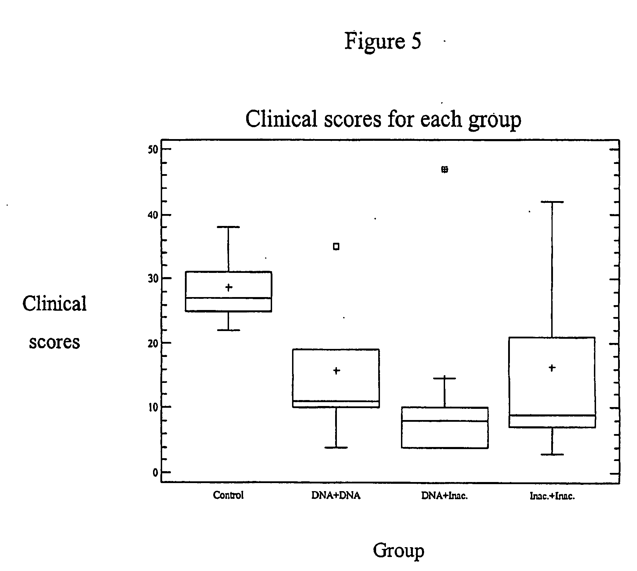

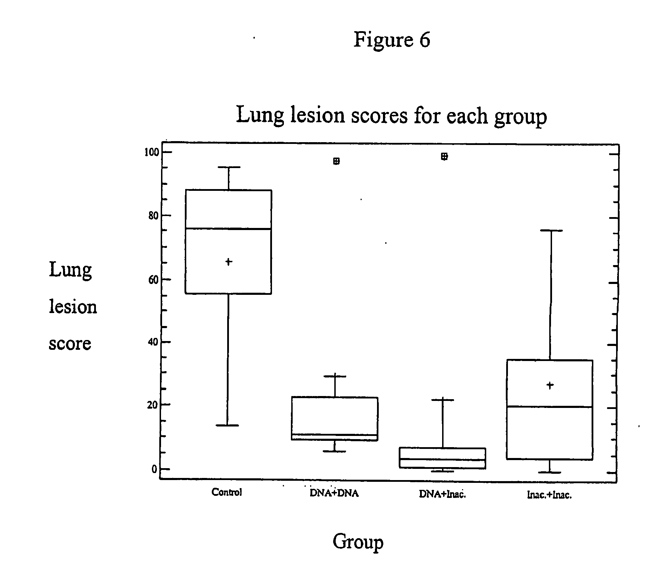

Nucleotide sequences encoding the BRSV antigens which can be used in the present invention and various expression vector constructs are given herein, e.g. in the accompanying examples, and in FR-A1-2751229, such as in Examples 9 and 10, and in Figures 5 and 6 (see also U.S. Patent No. 6,376,473 and U.S. application Serial No. 10/085,519).

The DNA vaccine or immunogenic or immunological composition against BRSV as described above can advantageously include a veterinarily acceptable vehicle or excipient according to the above-discussion of gthe invention.

The DNA vaccine or immunogenic or immunological composition against BRSV as described above can also comprises an adjuvant according to above-discussion of the invention, advantageously DMRIE, even more advantageously, DMRIE-DOPE.

The DNA vaccine or immunogenic or immunological composition against BRSV as described above, adjuvanted or not, can comprises GM-CSF or an expression vector, advantageously a plasmid, expressing GM-CSF, with the GM-CSF being advantageously bovine GM-CSF.

The addition of bovine GM-CSF may be carried out by the incorporation of the bovine GM-CSF polypeptide into the vaccinal or immunogenic or immunological composition or advantageously by the insertion of the nucleotide sequence encoding the bovine GM-CSF into an *in vivo* expression vector, such as a plasmid. The nucleotide sequence encoding GM-CSF can be inserted into a second expression plasmid (e.g. pLF1032 Example 8), different from that (or those) into which the gene(s) encoding the BRSV antigen(s) is(are) inserted.

A nucleotide sequence encoding bovine GM-CSF is accessible from the GenBank database under the accession number U22385.

Advantageously, according to the description the DNA vaccine or immunogenic or immunological composition against BRSV, which may be formulated with DMRIE-DOPE, is composed of an expression plasmid (e.g. pSB108 Example 4.1.2) encoding the F antigen of BRSV optimized by the deletion of the fragment of the nucleotide sequence of F encoding the transmembrane domain and the cytoplasmic tail, optionally also with deletion of the upstream region described above (e.g. pPB449 Example 4.1.4), and of a second expression plasmid (e.g. pFC123 Example 4.3) encoding the native N protein of BRSV.

In anotherembodiment, the present description relates to vaccination or immunization against bovine parainfluenza virus type 3 (bPI-3).

The bPI-3 virus is a *Paramyxovirus,* also a member of the *Paramyxoviridae* family (Tsai et al., Infect. Immun., 1975, 11, 783-803).

Nucleotide sequences encoding the hemagglutinin and neuraminidase proteins (HN) and the fusion protein (F) of bPI-3 are known and accessible from the GenBank database under the accession number U31671. The DNA vaccine or immunogenic or immunological composition against bPI-3 may thus comprise a DNA plasmid comprising nucleotide sequence(s) encoding the HN and/or F proteins.

The DNA vaccine or immunogenic or immunological composition against bPI-3 as described above advantageously contains a veterinarily acceptable vehicle or excipient according to the above-discussion

The DNA vaccine or immunogenic or immunological composition against bPI-3 as described above can comprise an adjuvant according to the above-discussion , such as DMRIE, advantageously DMRIE-DOPE.

These embodiements may optionally further include (1) the addition of GM-CSF or an expression vector, advantageously a plasmid, expressing GM-CSF, or (2) the optimization of at least one bPI-3 antigen, or (3) the addition of GM-CSF or an expression vector, advantageously a plasmid, expressing GM-CSF and the optimization of at least one bPI-3 antigen. The GM-CSF is advantageously bovine GM-CSF. The addition of GM-CSF may be carried out as is described for BRSV and in the above-discussion

The optimization of the antigens derived from bPI-3 is carried out by substitution, of a "signal" sequence, for example, by the substitution of a bPI-3 antigen signal sequence with a heterologous signal sequence, such as the signal sequence of human tPA, e.g., the substitution of the signal sequence of hemagglutinin-neuraminidase (HN) of bPI-3 and/or of the fusion protein (F) of bPI-3 with the signal sequence of human tPA, and/or by the deletion of the DNA fragment encoding the transmembrane domain of HN and/or of F, and/or by the insertion of an intron, such as intron II of the rabbit beta-globin, advantageously upstream of the nucleotide sequence encoding HN and/or F. The deletion of the DNA fragment encoding the transmembrane domain of one of these proteins is adsvantageously accompanied by deletion of the cytoplasmic taiL The DNA vaccine or immunogenic or immunological composition against bPI-3 according to the descriptionmay therefore encode and express a single optimized PI-3 antigen (HN or F) or both (HN and F).

Nucleotide; sequences encoding the bPI-3 antigens which can be used in the presentdescriptionand various expression vector constructs are given herein, e.g. the accompanying examples, and in FR-A1-2751229, such as in Examples 14 and 15, and in Figures 10 and 11(see also U.S. Patent No. 6,376,473 and U.S. application Serial No. 10/085,519).

Advantageously, according to the description the DNA vaccine or immunogenic or immunological composition against bPI-3 comprises DMRIB-DOPE, and is composed of an expression plasmid (e.g. pLF1025 Example 7.1.2) encoding the HN antigen of bPI-3 optimized by the insertion of the signal sequence of the human tPA in place of the signal sequence ofHN, by the deletion of the fragment of the nucleotide sequence ofHN encoding the transmembrane domain and the cytoplasmic tail and by the insertion of intron II of the rabbit beta-globin gene upstream ofHN, and of a second expression plasmid (e.g. pLF1027 Example 7.2.2) encoding the F antigen of bPI-3 optimized by the insertion of the signal sequence of the human tPA in place of the signal sequence of F, by the deletion of the fragment of the nucleotide sequence encoding the transmembrane domain of F and the cytoplasmic tail and by the insertion of intron II of the rabbit beta-globin gene upstream of F.

In another emodiment, the present description provides vaccination or immunization against infectious bovine rhinotracheitis (IBR).

The virus responsible for infectious bovine rhinotrachitis is a bovine herpesvirus type 1 (BHV-1), a member of the *Alphaherpesvirinae* family (Babiuk L.A. et al., 1996, Vet. Microbiol., 53, 31-42). Nucleotide sequences encoding the glycoproteins gB, gC and gD are known and are accessible from the GenBank database under the accession number AJ004801. The DNA vaccine or immunogenic or immunological composition against BHV-1 may thus comprise DNA plasmid or plasmids comprising the nucleotide sequence(s) encoding the gB, gC and/or gD proteins.

The DNA vaccine or immunogenic or immunological composition against BHV-1 as described above advantageously contains a veterinarily acceptable vehicle or excipient according to the above-discussion.

The DNA vaccine or immunogenic or immunological composition against BHV-1 as described above can comprises an adjuvant according to the above-discussion such as DMRIE, advantageously DMRIE-DOPE.

Optionally, these embodiments may optionally further include (1) the addition of GM-CSF or of an expression vector, advantageously a plasmid, expressing GM-CSF, or (2) the optimization of at least one BHV-1 antigen, or (3) the addition of bovine GM-CSF or of an expression vector, advantageously a plasmid, expressing GM-CSF and the optimization of at least one BHV-1 antigen. GM-CSF is advantageously bovine GM-CSF. The addition of GM-CSF may be carried out as described for BRSV and in the above general discussion

The optimization of the antigens derived from BHV-1 is carried out by substitution, of a "signal" sequence of a BHV-1 antigen with a heterologous "signal" sequence, e.g., the human tPA signal sequence (GenBank accession number NM_000930); for instance, substitution of the sequence of the signal peptide of the glycoprotein gB and/or of the glycoprotein gC and/or of the glycoprotein gD with the human tPA signal sequence; and/or by the deletion of the DNA fragment encoding the transmembrane domain of gB and/or of gC and/or of gD. The deletion of the DNA fragment encoding the transmembrane domain of one of theses glycoproteins is advantageously accompanied by deletion of the cytoplasmic tail. The DNA vaccine or immunogenic or immunological composition against BHV-1 according to the description can therefore encode and express a single optimized BHV-1 antigen (gB, gC or gD) or two of them or all three, e.g., optimized gB, optimized gC and optimized gD.

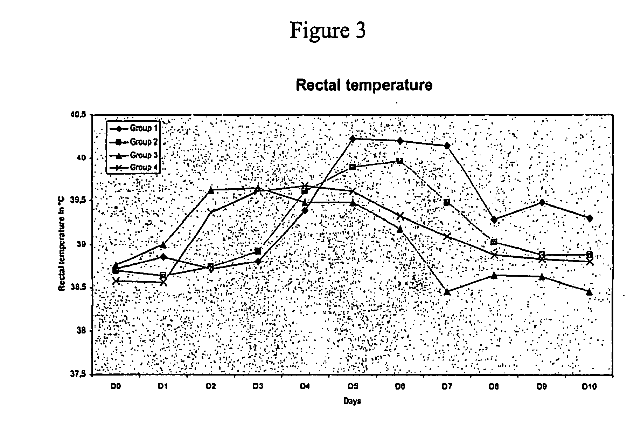

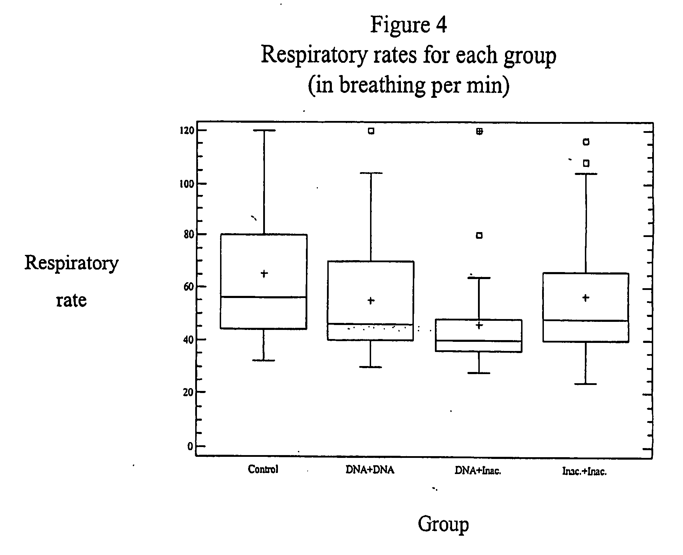

Nucleotide sequences encoding the BHV-1 antigens which can be used in the present description and various constructs of expression vectors are given herein, e.g. the accompanying examples, and in FR-A1-2751229, such as in Examples 7 and 8, and in Figures 3 and 4 (see also U.S. Patent No. 6,376,473 and U.S. application Serial No. 10/085,519).

Advantageously, according to the description the DNA vaccine or immunogenic or immunological composition against BHV-1 is formulated with DMRIE-DOPE, and is composed of an expression plasmid (e.g. pPB281, Example 3.1.2) encoding the BHV-1 gB antigen optimized by the deletion of the fragment of the nucleotide sequence encoding the transmembrane domain and the cytoplasmic tail, of a second expression plasmid (e.g. pPB292, Example 3.2.2) encoding the BHV-1 gC antigen optimized by the deletion of the fragment of the nucleotide sequence encoding the transmembrane domain and the cytoplasmic tail, and of a third expression plasmid (e.g. pPB284, Example 3.3.2) encoding the BHV-1 gD antigen optimized by the deletion of the fragment of the nucleotide sequence encoding the transmembrane domain and the cytoplasmic tail.

In yet another embodiment the present description provides vaccination or immunization against the BVDV.

The BVDV virus is a pestivirus of the *Flaviviridae* family. It is universally distributed in bovine populations and manifests itself by fetal malformations, abortions or clinical respiratory (mucosal disease) and enteric (bovine viral diarrhea) symptoms.

The BVDV viruses are distinguishable by the seriousness of the clinical signs and two groups have been formed, the BVDVs type 1 (inapparent or mild clinical signs) and those of type 2 (acute clinical signs, hemorrhage, high morbidity, high mortality) (Dean H.I. and Leyh R., 1999, Vaccine, 17, 1117-1124).

When a BVDV type is not clearly specified, this virus is understood to be type 1 or type 2.

The BVDV is an enveloped single-stranded RNA virus composed of a single gene encoding a polyprotein which, after cleavage, gives several well-individualized proteins, e.g., the E0 protein (gp48) and the E2 protein (gp53) (Vassilev VB. et al., 1997, J. Virol., 71, 471-478).

Nucleotide sequences encoding the E0-E2 polyproteins are known and accessible from the GenBank database under the accession number M96687 for BVDV-1 and AF145967 for BVDV-2. The DNA vaccine or immunogenic or immunological composition against BVDV may thus comprise DNA plasmid or plasmids containing nucleotide sequence(s) encoding the E0 and/or E2 proteins.

The DNA vaccine or immunogenic or immunological composition against BVDV as described above advantageously contain a veterinarily acceptable vehicle or excipient according to the above-discussion.

The DNA vaccine or immunogenic or immunological composition against BVDV as described above can comprise an adjuvant according to the above-discussion such DMRIE, advantageously DMRIE-DOPE.

These two embodiments may further include (1) the addition of bovine GM-CSF or of an expression vector, advantageously plasmid expressing GM-CSF, or (2) the optimization of at least one BVDV antigen, or (3) the addition of bovine GM-CSF or of an expression vector, advantageously plasmid expressing GM-CSF and the optimization of at least one BVDV antigen. GM-CSF is advantageously bovine GM-CSF. The addition of GM-CSF may be carried out as is described for BRSV and as described in the above general discussion.

The optimization of the antigens derived from BVDV is carried out by the addition of a "signal" sequence, such as a heterologous signal sequence, advantageously the human tPA signal sequence, advantageously upstream of the nucleotide sequence encoding the E0 protein of BVDV and/or the E2 protein of BVDV, and/or by the deletion of the DNA fragment encoding the transmembrane domain of E2, and/or by the insertion of an intron, such as intron II of the rabbit beta-globin, advantageously upstream of the nucleotide sequence encoding E0 and/or E2. The DNA vaccine or immunogenic or immunological composition against BVDV according to thedescriptionmay therefore encode and express a single optimized BVDV antigen (E0 or E2) or both (E0 and E2).

Nucleotide sequences encoding the BVDV antigens which can be used in the present description and various constructs of expression vectors are given herein, e.g. the accompanying examples, and in FR-A1-2751229, such as in Example 13, and in Figure 9 (see also U.S. Patent No. 6,376,473 and U.S. application Serial No. 10/085,519).

Advantageously, according to the description the DNA vaccine or immunogenic or immunological composition against BVDV is formulated with DMRIE-DOPE, and is composed of an expression plasmid (e.g. pLF1029 Example 5.1.2, pLF1031 Example 6.2.2) encoding the E0 antigen of BVDV optimized by the insertion of the signal sequence of the human tPA upstream of E0 and by the insertion of intron II of the rabbit beta-globin gene upstream of B0, and of a second expression plasmid (e.g. pLF1021 Example 5.22, pLF1023 Example 6.1.2) encoding the E2 antigen of BVDV optimized by the insertion of the signal sequence of the human tPA upstream of E2, by the deletion of the fragment of the nucleotide sequence encoding the transmembrane domain of E2 and the cytoplasmic tail and by the insertion of intron II of the rabbit beta-globin gene upstream of E2.

A mixture of plasmids can be advantageously produced and employed in the practice of the description e.g., the DNA vaccine or immunogenic or immunological composition can comprise a mixture of plasmids. The mixture may comprise at least two expression plasmids, each expressing a different immunogen (E0 or E2) and/or obtained from a different type of BVDV (BVDV-1 or BVDV-2). A mixture can comprise four plasmids:one expressing BVDV-1 E0, one expressing BVDV-1 E2, one expressing BVDV-2 E0 and one expressing BVDV-2 E2.

In an embodiment, the boost is done with an inactivated or attenuated or subunit vaccine or immunogenic or immunological composition. Inactivated or attenuated or subunit vaccines are available to the person skilled in the art (e.g. see Ellis et al., J. Am. Vet. Med. Assoc., 2001, 218(12), 1973-1980 for BRSV; GB Patent No. 1,131,851 for bPI-3; U.S. Patent No. 5,676,951 and Published U.S. Application No. 2002/0187929 for BHV; U.S. Patent No. 6,291,228 for BVDV). In addition one may use any commercial inactivated or attenuated or subunit vaccines, like BAR VAC® RS (Boehringer) for BRSV, VIROBOV H® (Merial) for bPI-3, IFFAVAX® I.B.R (Merial) for BHV, BOVILIS BVD® (Intervet) or MUCOBOVIN® (Merial) for BVDV.

Advantageously, the inactivated or attenuated or subunit vaccine or immunogenic or immunological composition comprises an adjuvant, e.g., an adjuvant as herein discussed.

In another embodiment, the boost is done with a recombinant vaccine or immunogenic or immunological composition which comprises an *in vivo* expression vector, such as a poxvirus, an adenovirus or a herpesvirus.

The expression vector comprises and expresses a nucleotide sequence encoding an immunogen from a bovine pathogen such as BRSV, bPI-3, BHV-1 and BVDV. The vector comprises and expresses at least one immunogen in common with the DNA vaccine or immunogenic or immunological composition. For these immunogens and the corresponding nucleotide sequences coding for these immunogens, reference is made to the herein description in relation with DNA vaccine or immunogenic or immunological composition. The nucleotide sequences may also be modified and improved as described herein.

Specific, non-limiting examples include recombinant poxvirus, including avipox viruses, such as canarypox virus (U.S. Patent No. 5,505,941) and vaccinia viruses (U.S. Patent No. 4,603,112), such as attenuated vaccinia virus such as NYVAC (see U.S. Patent No. 5,494,807) or Modified Vaccinia virus Ankara (MVA, Stickl H. and Hochstein-Mintzel V., Munch. Med Wschr. 113:1149-1153, 1971; Sutter G. et al., Proc. Natl. Acad Sci. U.S.A. 89:10847-10851, 1992; Carroll M. W. et al., Vaccine 15(4):387-394, 1997; Stittelaar K. J. et al., J. Virol. 74(9):4236-4243, 2000; Sutter G. et al., Vaccine 12(11):1032-1040, 1994). When avipox viruses are used, dovepox viruses, canarypox viruses (U.S. Patent No. 5,756,103) and fowlpox viruses (U.S. Patent No. 5,766,599) may be employed, such as attenuated viruses canarypox viruses and attenuated fowlpox viruses, for instance ALVAC and TROVAC. For recombinant canarypox virus vectors, the insertion sites may be the ORFs C3, C5 or C6. When the expression vector is a poxvirus, the heterologous polynucleotide can be inserted under the control of a poxvirus specific promoter, such as the vaccinia virus 7.5kDa promoter (Cochran et al., J. Virology 54:30-35, 1985), the vaccinia virus I3L promoter (Riviere et al., J. Virology 66:3424-3434, 1992), the vaccinia virus HA promoter (Shida, Virology 150:451-457, 1986), the cowpox virus ATI promoter (Funahashi et al., J. Gen. Virol. 69:35-47, 1988), the vaccinia virus H6 promoter (Taylor et al., Vaccine 6:504-508, 1988; Guo et al., J. Virol. 63:4189-4198, 1989; Perkus et al., J. Virol. 63:3829-3836, 1989).

Other useful viral vectors include herpesvirus or adenovirus vectors. Specific, non-limiting examples include bovine herpesvirus (BHV) or bovine adenovirus (BAV) as a vector (for example, see Published European Application No. EP 0.663.403; Published PCT Application No. WO 98/59063). For BHV, the insertion sites may be the thymidine kinase gene, in the gE, or in the gI (see Published PCT Application No. WO 92/21751). For BAV, the insertion sites may be the E2 region or in the E4 region (see U.S. Patent No. 6,451,319; U.S. Patent No. 6,319,716). In BHV or BAV vectors the insert (heterologous nucleic acid molecule encoding the immunogen, antigen or epitope of interest, e.g., of a bovine pathogen, such as that which is expressed by the DNA vaccine or immunogenic composition) is, in general, under the control of (or operably linked to) a promoter. The promoter may be of viral or cellular origin. The cytomegalovirus (CMV) early promoter (CMV-IE promoter), including the promoter and enhancer, may used. The CMV-IE promoter can be of human or murine origin, or optionally of other origin such as rat or guinea pig (see EP 0260148; EP 0323597; WO 89/01036; Pasleau et al., Gene 38:227-232, 1955; Boshart M. et al., Cell 41:521-530,1985); *see also* discussion above (concerning promoters for use in DNA plasmids). Functional fragments of the CMV-IE promoter may also be used (WO 98/00166); *see also* discussion above (concerning promoters for use in DNA plasmids). The SV40 virus early or late promoter and the Rous Sarcoma virus LTR promoter may also be used. Other promoters include but are not limited to, a promoter of the cytoskeleton gene, such as the desmin promoter (Kwissa M. et al., Vaccine 18(22):2337-2344, 2000), or the actin promoter (Miyazaki J. et al., Gene 79(2):269-277, 1989). Advantageously the promoter is a CMV-IE promoter.

The inactivated or attenuated or subunit vaccine or immunogenic or immunological composition, or the recombinant vaccine or immunogenic or immunological composition can be supplemented with an adjuvant such as fMLP (N-formyl-methionyl-leucyl-phenylalanine; U.S. Patent No.: 6,017,537) and/or acrylic acid or methacrylic acid polymer and/or a copolymer of maleic anhydride and of alkenyl derivative. The acrylic acid or methacrylic acid polymers can be cross-linked, e.g., with polyalkenyl ethers of sugars or of polyalcohols. These compounds are known under the term "carbomer" (Pharmeuropa, Vol. 8, No. 2, June 1996). A person skilled in the art may also refer to U.S. Patent No.2,909,462 which discusses such acrylic polymers cross-linked with a polyhydroxylated compound containing at least 3 hydroxyl groups: in one embodiment, a polyhydroxylated compound contains not more than 8 hydroxyl groups; in another embodiment, the hydrogen atoms of at least 3 hydroxyls are 34 replaced with unsaturated aliphatic radicals containing at least 2 carbon atoms; in other embodiments, radicals contain from about 2 to about 4 carbon atoms, e.g., vinyls, allyls and other ethylenically unsaturated groups. The unsaturated radicals can themselves contain other substituents, such as methyl. The products sold under the name Carbopol® (Noveon Inc., Ohio, USA) are particularly suitable for use as an adjuvant They are cross-linked with an allyl sucrose or with allylpentaerythritol, as to which, mention is made of the products Carbopol® 974P, 934P, and 971P.

As to the copolymers of maleic anhydride and of alkenyl derivative, mention is made of the EMA® products (Monsanto) which are copolymers of maleic anhydride and of ethylene, which may be linear or cross-linked, for example cross-linked with divinyl ether. Also, reference may be made to J. Fields et al., Nature 186:778-780,1960. Generally, the acrylic acid or methacrylic acid polymers, such as the carbomers, and the copolymers of maleic anhydride and of alkenyl derivative, such as the EMA® products, are formed from units based on the following formula: 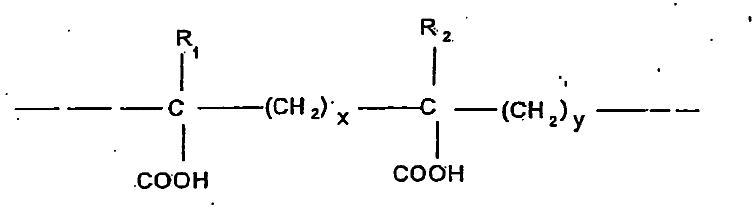 in which:
- R₁ and R₂, which may be identical or different, represent H or CH₃
- x = 0 or 1, advantageously, x =1
- y = 1 or 2, with x + y = 2.

For the EMA® products, x = 0 and y = 2. For the carbomers, x = y =1.

The dissolution of these polymers in water leads to an acid solution, which is neutralized to physiological pH, in order to give the adjuvant solution into which the immunogenic composition or the vaccine itself is incorporated. The carboxyl groups of the polymer are then partly in COO⁻ form.

Advantageously, a solution of adjuvant, e.g., carbomer, is prepared in distilled water, for example, in the presence of a salt such as sodium chloride; the solution obtained is at acidic pH. This stock solution is diluted by adding it to the desired quantity (for obtaining the desired final concentration), or a substantial part thereof, of water containing a salt such as NaCl, advantageously physiological saline (NaCL 9 g/l) all at once or in several portions with concomitant or subsequent neutralization (pH 7.3 to 7.4). The stock solution is neutralized with a base such as NaOH. This solution of adjuvant at physiological pH is used as it is for mixing with the immunogenic composition or with the vaccine, which may be especially stored in freeze-dried, liquid or frozen form.

The polymer concentration in the final vaccine composition can be from about 0.01% to about 1.5% W/V. The final vaccine composition can be from about 0.05 to about 1% W/V. The final vaccine composition can be from about 0.1 to about 0.4% W/V.

The inactivated or attenuated or subunit or recombinant vaccine or immunogenic or immunological composition can also be formulated in the form of an oil-in-water emulsion. The oil-in-water emulsion can be based, for example, on light liquid paraffin oil (European Pharmacopea type); isoprenoid oil such as squalane, squalene, EICOSANE^{™} or tetratetracontane; oil resulting from the oligomerization of alkene(s), e.g., isobutene or decene; esters of acids or of alcohols containing a linear alkyl group, such as plant oils, ethyl oleate, propylene glycol di(caprylate/caprate), glyceryl tri(caprylate/caprate) or propylene glycol dioleate; esters of branched fatty acids or alcohols, e.g., isostearic acid esters. The oil advantageously is used in combination with emulsifiers to form the emulsion. The emulsifiers can be nonionic surfactants, such as esters of sorbitan, mannide (e.g., anhydromannitol oleate), glycerol, polyglycerol, propylene glycol, and oleic, isostearic, ricinoleic, or hydroxystearic acid, which are optionally ethoxylated, and polyoxypropylene-polyoxyethylene copolymer blocks, such as the Pluronic® products, e.g., L121. In one specific, non-limiting example, the oil is provided in an amount between about 1 and about 60%. The oil can be in an amount between about 5 and about 30%. The adjuvant can be a mixture of emulsifier(s), micelle-forming agent, and oil such as that which is available under the name Provax® (IDEC Pharmaceuticals, San Diego, CA).

The DNA plasmid, the inactivated or attenuated or subunit or the expression, e.g., viral, vector according to this disclosure can be preserved and/or conserved and stored either in liquid form, e.g., at low temperature, e.g. at about 5°C, or in lyophilised or freeze-dried form, e.g., in the presence of a stabilizer. Freeze-drying can be according to well-known standard freeze-drying procedures. The pharmaceutically acceptable stabilizers may be SPGA (sucrose phosphate glutamate albumin) (Bovamik et al., J. Bacteriology 59:509, 1950), carbohydrates (*e.g.,* sorbitol, mannitol, lactose, sucrose, glucose, dextran, trehalose), sodium glutamate (Tsvetkoy T et al., Cryobiology 20(3):318-23, 1983 ; Israeli E et al., Cryobiology 30(5):519-23, 1993), proteins such as peptone, albumin or casein, protein containing agents such as skimmed milk (Mills CK et al., Cryobiology 25(2):148-52, 1988; Wolff E et al., Cryobiology 27(5):569-75, 1990), and buffers (e.g., phosphate buffer, alkaline metal phosphate buffer). An adjuvant and/or a vehicle or excipient may be used to make soluble the freeze-dried preparations.

Another aspect of the presentdescriptionis the use of plasmids containing and expressing *in vivo* in a bovine at least one immunogen from a bovine pathogen, e.g., BRSV, bPI-3, BHV-1 or BVDV, for the preparation of a DNA vaccine, e.g., for use in a prime-boost method of the description and/or for a kit for a prime-boost method of the descriptionand/or to induce an immune response in a young bovine, e.g., calve, which have or may have maternal antibodies against the bovine pathogen.

Advantageously, the DNA vaccine is to be administered (and is administered) to the young animal (bovine) from calving up to and including about 12 weeks of age, such as from calving up to and including 6 weeks of age, for instance, from calving up to and including 4 weeks of age, e.g., from calving up to and including 3 weeks of age.

More advantageously, the DNA vaccine is intended to induce (and induces) a priming immune response specific for the expressed immunogen or a "DNA induced immune response" (such as a gamma-interferon+ (IFN_{γ}+) memory T cell response specific for the expressed immunogen), which is boostable (can be boosted), by a subsequent administration (boost) of an inactivated or attenuated or subunit vaccine or a recombinant or modified vector (e.g., viral, bacterial) vaccine or immunogenic composition comprising a vector, e.g., viral vector, such as a live recombinant poxvirus, containing and expressing *in vivo* at least the same immunogen(s), antigen(s) or epitope(s) as that expressed by the DNA vaccine.

The boost administration may be administered from about 2 weeks to about 5 months after the priming administration, such as from about 3 to about 6 weeks after the priming administration, and advantageously about 4 weeks after the priming administration. A second administration of the boost vaccine or immunological or immunogenic composition may occur, for instance, when the calve is transferred to a finishing unit

In another aspect, the present description involves the use of a bovine pathogenic agent (including a fragment thereof), such as BRSV, bPI-3, BHV-1 or BVDV, for the preparation of a priming vaccine based on plasmids (DNA vaccine or immunogenic or immunological composition) containing and expressing *in vivo* in a bovine (e.g., cow, bull, calve, cattle) a nucleic acid molecule encoding at least one immunogen, antigen or epitope from the pathogenic agent, and for the preparation of a second vaccine (a boost vaccine or immunogenic or immunological composition) comprising the pathogenic agent in an inactivated form, or in an attenuated form, or wherein the second vaccine comprises a subunit (isolated protein, antigen, immunogen or epitope) of the pathogenic agent or wherein the second vaccine comprises a recombinant or modified expression vector (e.g., viral or bacterial or yeast vector), such as a live recombinant poxvirus, advenovirus, or herpesvirus, advantageously a poxvirus, that contain(s) and express(es) *in vivo* nucleic acid molecule(s) encoding at least the immunogen(s), antigen(s) or epitope(s) of the pathogenic agent, including such immunogen(s), antigen(s) or epitope(s) expressed by the plasmid-based vaccine. Here, "use of a pathogenic agent" encompasses the use of the pathogen to clone the nucleotide sequence encoding the immunogen, antigen or epitope, as well as the use of the pathogenic agent to isolate therefrom the immunogen, antigen or epitope (e.g., for the subunit or to sequence the same to ascertain a coding sequence therefore for preparation of the DNA plasmid or the recombinant or modified expression vector), as well as the use of the pathogenic agent to prepare the inactivated or attenuated vaccine or immunogenic composition. Thus, another aspect of the present description is the use of a nucleotide sequence coding for at least one immunogen, antigen or epitope of a bovine pathogenic agent, such as BRSV, bPI-3, BHV-1 or BVDV, for the preparation of the priming vaccine based on plasmid (DNA vaccine or immunogenic or immunological composition) and/or the second vaccine (or immunogenic or immunological composition) that contains the modified or recombinant expression vector. The subunit, inactivated or attenuated vaccine or immunogenic composition advantageously contains the immunogen, antigen or epitope expressed by the DNA vaccine or immunogenic or immunological composition; and, the recombinant or modified expression vector advantageously expresses the immunogen, antigen or epitope expressed by the DNA vaccine or immunogenic or immunological composition. The plasmid-based vaccine is intended to be administered to a bovine first (such as to a young calve which has or may have maternal antibodies against the bovine pathogen), and the inactivated or attenuated or subunit or recombinant or modified expression vector vaccine is intended to be administered after the DNA vaccine and to the same bovine, to boost the immune reponse against the immunogen, antigen or epitope (for instance, at the time the inactivated or attenuated or subunit or recombinant or modified expression vector vaccine is administered, the calve has developed a specific priming immune response against the immunogen(s), antigen(s) or epitope(s), such as a specific DNA vaccine immune response or "DNA vaccine induced" immune response against the immunogen(s), antigen(s), or epitope(s), e.g., the IFN_{γ}+ memory T cell response specific for the expressed immunogen, antigen or epitope; and the inactivated or attenuated or subunit or recombinant or modified expression vector vaccine induces an immune response against the bovine pathogen including against at least one of the the immunogen(s), antigen(s) or epitope(s) expressed by the DNA vaccine).

Accordingly another aspect of the present description is the use of a bovine pathogenic agent, such as BRSV, bPI-3, BHV-1 or BVDV, to prepare an inactivated or attenuated or subunit vaccine or immunogenic composition to vaccinate or immunize a bovine against the pathogenic agent, wherein the bovine (e.g., a young calve which has or may have maternal antibodies against the bovine pathogen) has previously been immunized with a DNA vaccine that expresses *in vivo* at least one immunogen, antigen or epitope from the same pathogenic agent and has developed a specific priming immune response against the immunogen(s), antigen(s) or epitope(s), such as the "DNA vaccine induced" immune response, more advantageously the IENγ+ memory T cell response specific for the expressed immunogen, antigen or epitope.

Likewise, another aspect of the present description is the use of a recombinant or modified expression vector, e.g., a viral vector, such as a poxvirus vector, that comprise(s) and express(es) *in vivo* at least one nucleotide sequence coding for at least one immunogen, antigen or epitope from a bovine pathogenic agent, such BRSV, bPI-3, BHV-1 or BVDV, to prepare a recombinant or modified expression vector (e.g., a live recombinant or modified vector) vaccine or immunogenic composition to vaccinate a bovine against the pathogenic agent, wherein the bovine (such as a young calve which has or may have maternal antibodies against the bovine pathogen) has previously been immunized with a DNA vaccine that expresses *in vivo* at least the same immunogen(s), antigen(s) or epitope(s) and has developed a priming immune response against the immunogen(s), antigen(s) or epitope(s), such as the "DNA vaccine induced" immune response, more advantageously, the IFNγ+ memory T cell response specific for the expressed immunogen, antigen or epitope.

The DNA vaccine is advantageously administered to the young animal (bovine) from calving up to and including about 12 weeks of age, such as from calving up to and including about 6 weeks of age, for instance, from calving up to and including 4 weeks of age, e.g., from calving up to and including 3 weeks of age. The inactivated or attenuated or subunit or recombinant or modified expression vector vaccine or immunogenic composition is intended to be administered from about 2 weeks to about 5 months after the priming administration, such as from about 3 to 6 weeks after, and advantageously about 4 weeks after. A second administration of the boost vaccine or immunological or immunogenic composition may occur, such as when the calve is transferred to a finishing unit.

The inactivated or the recombinant or modified expression vector vaccine or immunogenic composition is advantageously employed in the practice of the description The inactivated or attenuated or subunit or recombinant or modified expression vector vaccine or immunogenic composition can be as described herein and can advantageously comprise an adjuvant. To avoid repitition, it is generally mentioned that discussion elsewhere in this description may be applied to the herein "use" aspects of the invention.

The method and uses of the description may combine immunization or vaccination against more than one bovine pathogen, and the methods and uses of the description may encompass any combination immunization or vaccination against 2, 3 or 4 of the particular herein mentioned pathogens. This may be performed by concomitant or successive administration of the vaccines or immunogenic or immunologic composition against the pathogens. This may also involve mixtures of the corresponding immunogens or vaccines or immunogenic or immunologic composition. This may also involve plasmids or vectors comprising and expressing nucleic acid molecules encoding immunogens, antigens or epitopes of two or more or several pathogens. Accordingly, another aspect of the description is multivalent. DNA vaccines or immunogenic or immunological compositions.

Still another aspect of the description is a kit containing a first vaccine or immunogenic or immunological composition which comprises a DNA vaccine or immunogenic or immunological composition according to the description and a second vaccine or immunogenic or immunological composition comprising an inactivated, attenuated live, subunit, advantageously inactivated vaccine or ! immunogenic or immunological composition, or recombinant or modified *in vivo* expression vector vaccine or immunogenic or immunological composition, wherein the subunit contains an immunogen or antigen or epitope expressed by the first vaccine and the modified vector vaccine or immunogenic or immunological composition expresses an immunogen, antigen or epitope expressed by the first vaccine, and the attenuated vaccine or immunogenic or immunological composition expresses or presents an immunogen, antigen or epitope expressed by the first vaccine, and the inactivated vaccine or immunogenic or immunological composition presents an immunogen, antigen or epitope expressed by the first vaccine. The vaccine(s) or composition(s) are advantageously in separate containers. The separate containers can be packaged together. The kit can contain instructions for prime-boost administration according to the description.

The quantity of DNA used in the vaccines and compositions according to the present description is advantageously between about 1 µg and about 1000 µg, and such as between about 50 µg and about 500 µg, for a given plasmid. Persons skilled in the art possess the competence necessary to precisely define the effective dose of DNA to be used for each vaccination protocol, from this disclosure and the knowledge in the art.

The dose volumes may be between about 0.2 and about 5 ml, advantageously between about 1 and about 3 ml.

The DNA vaccines and compositions according to the descriptionmay be administered, in the context of th vaccination method of the description by various routes of administration proposed in the art for polynucleotide vaccination and by means of known techniques of administration.

According to a mode of the description, the DNA vaccines or compositions according to the description are administered by the intramuscular route, the subcutaneous route or with the aid of a needleless injector such as the Biojector 2000 (Bioject Inc., Portland OR, USA), advantageously by the intradermal route. For more details on administration to a bovine via the intradermal route using a needleless injector, one may refer to US-A-6,451,770.

For the boost administration with a recombinant or modified *in* vivo expression vector vaccine or immunogenic or immunological composition (e.g., a recombinant virus such as a recombinant herpesvirus, adenovirus, or poxvirus composition, advantageously a recombinant poxvirus composition, such as a recombinant vaccinia, avipox, canarypox fowlpox virus composition, advantageously an ALVAC, TROVAC, or NYCAC composition), the route of administration can be intradermal (ID), intramuscular (IM), subcutaneous (SC), intravenous, oral or nasal. This administration can be made with a syringe and a needle or with a needleless injector. The dosage is advantageously from about 10³ pfu to about 10⁹ pfu per recombinant vector. When the vector is a canarypox virus, the dosage is advantageously from about 10⁵ pfu to about 10⁹ pfu, e.g. from about 10⁶ pfu to about 10⁷ pfu. The volume of needleless injector doses could be between about 0.1 ml and about 0.5 ml, e.g. about 0.25 ml. For injection with a syringe and a needle, the volumes advantageously can be from about 0.5 to about 5 ml, e.g. about 1 to about 3 ml. The dosage can be as mentioned herein.

For the boost administration with an inactivated, attenuated or subunit vaccine or immunogenic or immunological compositions, the route of administration can be intradermal (ID), intramuscular (IM), subcutaneous (SC), intravenous, oral or nasal. This administration can be made with a syringe and a needle or with a needleless injector. The volume of needleless injector doses advantageously can be between about 0.1 ml and about 0.5 ml, e.g. about 0.25 ml. For injection with a syringe and a needle, the volumes advantageously can be from about 0.5 to about 5 ml, e.g. from about 1 to about 3 ml.

The invention will now be further described and illustrated by way of the following, non-limiting examples.

### EXAMPLES

For each of the pathogens considered, each gene encoding the principal antigens (native form and modified form) was the subject of a particular construction in a eukaryotic expression plasmid. The secreted forms of the antigens were obtained by deletion of the fragments of genes encoding the transmembrane and cytoplasmic domains. In all cases, the transmembrane domains of the proteins were identified on the basis of the hydropathy profiles (on MacVector 6.5) of the corresponding protein sequences.

### Example 1: Molecular biology methods

### 1.1 Extraction of viral genomic DNA

Viral suspensions were treated with proteinase K (100 mg/ml final) in the presence of sodium dodecyl sulphate (SDS) (0.5% final) for 2 hours at 37°C. The viral DNA was then extracted with the aid of a phenol/chloroform mixture, and then precipitated with two volumes of absolute ethanol at -20°C for 16 hours and then centrifuged at 10,000 g for 15 minutes at 4°C. The DNA pellets were dried, and then taken up in a minimum volume of sterile ultrapure water.

### 1.2 Isolation of viral genomic RNA

The genomic RNA of each virus was extracted using the "guanidinium thiocyanate/phenol-chlorofarm" technique described by P. Chomczynski and N. Sacchi (Anal. Biochem. 1987.162.156-159).

### 1.3 Molecular biology techniques

All the constructions of plasmids were carried out using the standard molecular biology techniques described by Sambrook et al. (Molecular Cloning. A Laboratory Manual. 2nd Edition. Cold Spring Harbor Laboratory, Cold Spring Harbor, New York, 1989). All the restriction fragments used for the present description were isolated with the aid of the "Geneclean" kit (BIO101 Inc., La Jolla, CA). For all the constructs, the cloned DNA fragments, as well as the junctions with the expression vector, were sequenced by the Sanger method (Sambrook *et al*.,, 1989).

### 1.4 PCR and RT-PCR

The oligonucleotides specific to the genes or gene fragments cloned were synthesized, some of them containing, in some cases, at their 5' end, restriction sites facilitating the cloning of the amplified fragments. The reverse transcription (RT) reactions and the chain reaction (PCR) were carried out according to standard techniques (Sambrook *et al.,* 1989).

### 1.5 Large-scale purification of plasmids

The production, on the scale of about ten mg, of purified plasmids entering into the vaccinal compositions was carried out by the caesium chloride-ethidium bromide gradient method (Sambrook *et al.,* 1989).

### Example 2: Basic plasmid constructs

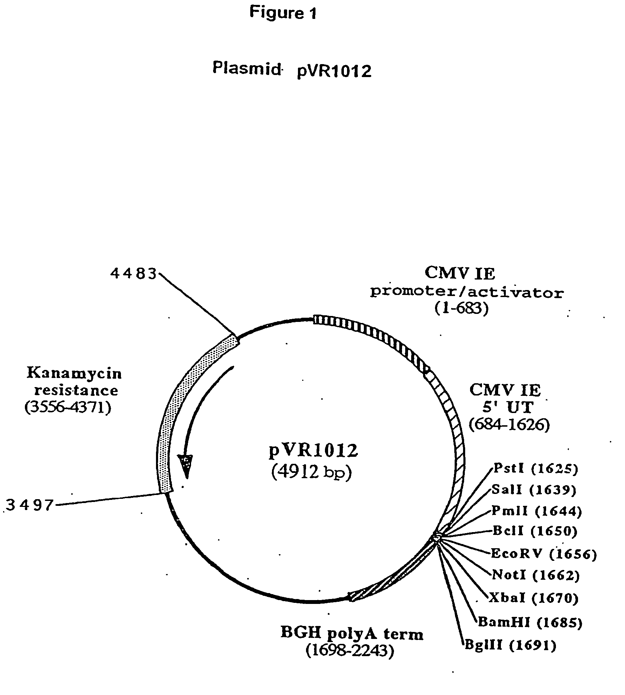

The eukaryotic expression plasmid pVR1020 (C.J. Luke et al. J. of Infectious Diseases, 1997, 175, 95-97), derived from the plasmid pVR1012 (Figure No. 1, Figure 1 and Example 7 of WO-A-9803199), contains the coding phase of the signal sequence of the human tissue plasminogen activator (tPA).

A plasmid pVR1020 is modified by BamHI-BgIII digestion and insertion of a sequence containing several cloning sites (BamHI, NotI, EcoRI, XbaI, PmII, PstI, BgIII) and resulting from the pairing of the following oligonucleotides:
PB326 (40 mer) (SEQ ID NO 1)
   5' GATCTGCAGCACGTGTCTAGAGGATATCGAATTCGCGGCC 3' and
PB329 (40 mer) (SEQ ID NO 2)
   5' GATCCGCGGCCGCGAATTCGATATCCTCTAGACACGTGCT 3'.

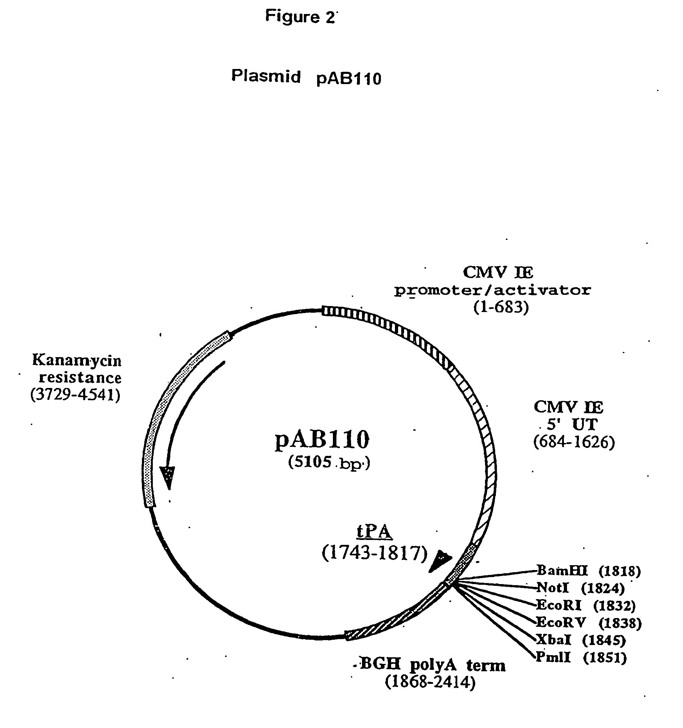

The vector thus obtained, having a size of about 5105 base pairs (or bp), is called pAB110 (Figure No. 2).

Intron II of the rabbit β-globin gene is cloned into the vector pCRII (Invitrogen, Carlsbad, CA, USA) after production of the corresponding DNA fragment by PCR with the aid of the following oligonucleotides:
SB090 (20 mer) (SEQ ID NO 3)
   5' TTGGGGACCCTTGATTGTTC 3' and
SB091 (21 mer) (SEQ ID NO 4)
   5' CTGTAGGAAAAAGAAGAAGGC 3'
using as template the genomic DNA of rabbit peripheral blood cells. The resulting plasmid is designated pNS050.

The expression plasmid pAB 110 is modified by introducing the sequence of intron II of the rabbit globin gene into the SalI site situated upstream of the ATG of the signal peptide of tissue plasminogen activator (tPA). The sequence of intron II of the rabbit globin gene is amplified by polymerase chain reaction (PCR) from the plasmid pNS050 using the following oligonucleotide pair:
LF001 (30 mer) (SEQ ID NO 5)
   5' CTCCATGTCGACTTGGGGACCCTTGATTGT 3' and
LF002 (30 mer) (SEQ ID NO 6)
   5' CTCCATGTCGACCTGTAGGAAAAAGAAGAA 3'

The PCR product (573 base pairs or bp) is digested with SalI and cloned into the plasmid pAB 110 previously linearized with SalI, to generate the plasmid pLF999 of about 5678 bp.

### Example 3: Plasmids encoding the various forms of the bovine herpesvirus type 1 (BHV-1) antigens

Fragments of viral DNA containing the gB, gC and gD genes of the B901 strain of BHV-1 are isolated by digesting the viral genome with various restriction enzymes, by separating them by agarose gel electrophoresis and by analysing them by Southern blotting with the aid of probes corresponding to fragments of the gB, gC and gD genes of the ST strain of BHV-1 (Leung-Tack P. et al., Virology, 1994, 199,409-421). The BHV-1 Colorado strain [Cooper] (ATCC number VR-864) can also be used. The fragments thus identified are cloned into the vector pBluescript SK+ (Stratagene, La Jolla, CA, USA) and are at the origin of the clonings of the three genes into the expression vector pVR1012.

### 3.1 Plasmids encoding the various forms of BHV-1 gB

### 3.1.1 pPB280: gB gene (native form) cloned into the vector pVR1012

Two XhoI-XhoI fragments containing the 5' and 3' portions of the BHV-1 gB gene are identified by Southern blotting and cloned into the vector pBluescript SK+ (Stratagene, La Jolla, CA, USA) previously digested with XhoI. The plasmids thus obtained are designated pPB128 and pPB 117 respectively.

The plasmid pPB128, containing the 5' fragment of the gB gene, is digested with NotI and XhoI, generating a fragment of 1708 bp (fragment A).

The plasmid pPB117, containing the 3' portion of the gB gene, is digested with XhoI and StuI, generating a fragment of 1345 bp. The latter fragment is cloned into the vector pBluescript KS+ (Stratagene, La Jolla, CA, USA) previously digested with EcoRV and XhoI. The resulting plasmid is called pPB279. The plasmid pPB279 is then digested with XhoI and BamHI, generating a DNA fragment of 1413 bp (fragment B).

Fragments A and B are then cloned into a vector pBluescript KS+ digested with NotI and BamHI, generating plasmid pPB278 (about 6063 bp) and allowing the reconstitution of the BHV-1 gB gene.

The vector pPB278 then serves as template during a PCR reaction carried out with the following oligonucleotides:
PB234 (30 mer) (SEQ ID NO 7)
   5' TTGTCGACATGGCCGCTCGCGGCGGTGCTG 3' and
PB235 (21 mer) (SEQ ID NO 8)
   5' GCAGGGCAGCGGCTAGCGCGG 3'.

The PCR product (146 bp) is then digested with the restriction enzymes SalI and NheI.

The plasmid pPB278 is digested with NheI and BamHI. The fragment of 2728 bp thus obtained and the PCR fragment previously digested are ligated into the vector pVR1012 (Example 2) previously digested with SalI and BamHI, thus generating the plasmid pPB280, having a size of about 7742 bp.

The BHV-1 gB gene encodes a protein of 933 amino acids.

### 3.1.2 pPB281: gB gene (Δ[TM-Cter] form) cloned into the vector pVR1012

The truncated form (deleted for its transmembrane (TM) and carboxy-terminal (Cter) domains) of the BHV-1 gB gene is obtained by ligating into the plasmid pVR1012 (Example 2) predigested with SalI and BamHI, both a fragment having a size of 2234 bp obtained after digestion with SalI-PvuII of the plasmid pPB280 (Example 3.1.1) and a fragment of 56 bp obtained by pairing of the following oligonucleotides:
PB511 (52 mer) (SEQ ID NO 9)
   5'CTGCACGAGCTCCGGTTCTACGACATTGACCGCGTGGTCAAGACGGAC TGAG 3' and

PB512 (57 mer) (SEQ ID NO 10)
5' GATCCTCAGTCCGTCTTGACCACGCGGTCAATGTCGTAGAACCGGAGC
TCGTGCAG 3'.

The plasmid thus generated has a size of about 7154 bp and is called pPB281. The truncated gB gene of BHV-1 encodes a protein of 759 amino acids.

### 3.1.3 pSB115: gB gene (tPA Δ[TM-Cter] form) cloned into the vector pAB110

The tPA Δ[TM-Cter] form of the BHV-1 gB gene is amplified by PCR from the template pPB281 (Example 3.1.2) and with the aid of the following primers:
SB221 (39 mer) (SEQ ID NO 11)
   5'AAAATTTCGATATCCGCCGCGGGGCGACCGGCGACAACG 3' and
SB222 (33 mer) (SEQ ID NO 12)
   5' GGAAGATCTTCAGTCCGTCTTGACCACGCGGTC 3'

The amplification product (2088 bp) is digested with the enzymes EcoRV and BgIII and cloned into the vector pAB110 (Example 2) previously digested with EcoRV and BgIII, generating the plasmid pSB115, having a size of about 7154 bp.

The tPA Δ[TM-Cter] form of the gB gene encodes a glycoprotein of 729 amino acids, containing the extracellular domain of the BHV-1 gB glycoprotein.

### 3.2. Plasmids encoding the various forms of BHV-1 gC

### 3.2.1 pPB264: gC gene (native form) cloned into the vector pVR1012

A BamHI-HindIII fragment of 3.5 kb containing the complete BHV-1 gC gene is identifed by Southern blotting and cloned into the vector pBluescript SK+. The plasmid thus obtained is called pPB287.

The plasmid pPB287 is then digested with NcoI-BssSI. A digestion fragment having a size of 1492 bp is obtained. It is ligated with a synthetic DNA fragment obtained by the pairing of the following oligonucelotides:
PB507 (37 mer) (SEQ ID NO 13)
   5'TCGTGCCTGCGGCGCAAGGCCCGGGCGCGCCTGTAGT 3' and
PB508 (37 mer) (SEQ ID NO 14)
   5' CTAGACTACAGGCGCGCCCGGGCCTTGCGCCGCAGGC 3',
into the plasmid pLitmus 28 (New England Biolabs, Inc., Beverly, MA, USA) predigested with NcoI and XbaI, generating the intermediate plasmid pPB290.

The fragment of 1554 bp derived from the digestion of pPB290 with PstI and XbaI is cloned into the vector pVR1012 (Example 2) previously digested with PstI and XbaI, thus generating the plasmid pPB264, having a size of about 6427 bp. The BHV-1 gC gene encodes a protein of 508 amino acids.

### 3.2.2 pPB292: gC gene (Δ[TM-Cter] form) cloned into the vector pVR1012

The truncated form of the BHV-1 gC gene is obtained by ligating the following three DNA fragments into the vector pVR1012 (Example 2) previously digested with PstI and XbaI:
(a) a fragment of 1035 bp derived from the digestion of pPB264 (Example 3.2.1) with PstI and XhoI,
(b) a fragment of 350 bp derived from the digestion of pPB264 with XhoI and BanI and
(c) a synthetic fragment of 43 bp resulting from the pairing of the oligonucleotides PB513 and PB514.

These oligonucleotides are the following:
PB513 (43 mer) (SEQ ID NO 15)
   5'GCACCGCTGCCCGAGTTCTCCGCGACCGCCACGTACGACTAGT 3' and
PB514 (43 mer) (SEQ ID NO 16)
   5' CTAGACTAGTCGTACGTGGCGGTCGCGGAGAACTCGGGCAGCG 3'.

The plasmid having a size of about 6305 bp thus obtained is called pPB292. The truncated gC gene of BHV-1 encodes a protein of 466 amino acids.

### 3.2.3 pSB116: gC gene (tPA Δ[TM-Cter] form) cloned into the vector pAB110

The tPA Δ[TM-Cter) form of the BHV-1 gC gene is amplified by PCR from the template pPB292 (Example 3.2.2) and with the aid of the following primers:
SB223 (39 mer) (SEQ ID NO 17)
   5'AAAATTTCGATATCCCGGCGGGGGCTCGCCGAGGAGGCG 3' and
SB224 (32 mer) (SEQ ID NO 18)
   5' GGAAGATCTCTAGTCGTACGTGGCGGTCGCGG 3'

The amplification product (1362 bp) is digested with the enzymes EcoRV and BglII and cloned into the vector pAB110 (Example 2) previously digested with EcoRV and BglII, generating the plasmid pSB116, having a size of about 6404 bp.

The tPA Δ[TM-Cter] form of the gC gene encodes a glycoprotein of 479 amino acids, containing the extracellular domain of the BHV-1 gC glycoprotein.

### 3.3 Plasmids encoding the various forms of BHV-1 gD

### 3.3.1 pPB148: gD gene (native form) cloned into the vector pVR1012

A XhoI-XhoI fragment of 5 kb containing the BHV-1 gD gene is identified by Southern blotting and cloned into the vector pBluescript SK+ predigested with XhoI, generating the plasmid pPB147.

A fragment of 325 bp derived from the digestion of pPB147 with NdeI and BsrBI and a fragment of 943 bp derived from the digestion of pPB 147 with NdeI and StyI are then ligated into the vector pVR1012 (Example 2) predigested with EcoRV and XbaI, thus generating the plasmid pPB148, having a size of about 6171 bp. The BHV-1 gD gene encodes a protein of 417 amino acids.

### 3.3.2 pPB284: gD gene (Δ[TM-Cter] form) cloned into the vector pVR1012

The truncated gD gene of BHV-1 is obtained from a fragment obtained after PCR amplification carried out on the genomic DNA of the B901 strain of the BHV-1 virus previously digested with PstI and XbaI and with the aid of the following primer pair:
PB497 (33 mer) (SEQ ID NO 19)
   5' TTTCTGCAGATGCAAGGGCCGACATTGGCCGTG 3' and
PB498 (31 mer) (SEQ ID NO 20)
   5' TTTCTAGATTAGGGCGTAGCGGGGGCGGGCG 3'.

This PCR fragment is then cloned into the plasmid pVR1012 (Example 2) previously digested with PstI and XbaI, generating the plasmid pPB284, having a size of about 5943 bp. The truncated gD gene of BHV-1 encodes a protein of 355 amino acids.

### 3.3.3 pSB117: gD gene (tPA Δ[TM-Cter] form) cloned into the vector pAB110

The tPA Δ[TM-Cter] form of the BHV-1 gD gene is amplified by PCR from the pPB284 template (Example 3.3.2) and with the aid of the following primers:
SB225 (39 mer) (SEQ ID NO 21)
   5' AAAATTTCGATATCCCCCGCGCCGCGGGTGACGGTATAC 3' and
SB226 (33 mer) (SEQ ID NO 22)
   5' GGAAGATCTTTAGGGCGTAGCGGGGGCGGGCGG 3'.

The amplification product (1029 bp) is digested with the enzymes EcoRV and BglII and cloned into the vector pAB110 (Example 2) previously digested with EcoRV and BglII, generating the plasmid pSB117, having a size of about 6071 bp.

The tPA Δ[TM-Cter] form of the gD gene encodes a glycoprotein of 368 amino acids, containing the extracellular domain of the BHV-1 gD glycoprotein.

### Example 4: Plasmids encoding the various forms of the bovine respiratory sencitial virus (BRSV) antigens

The genes encoding the F and G antigens of the BRSV virus are obtained by RT-PCR from the viral RNA of the Snook strain (Thomas et al. Research in Vet. Science, 1982, 33, 170-182). The BRSV A 51908 strain (ATCC number VR-794) may also be used.

### 4.1 Plasmids encoding the various forms of BRSV-F

### 4.1.1 pSB107: F gene (native form) cloned into the vector pVR1012

The F gene of the Snook strain of BRSV is amplified by RT-PCR using the viral RNA as template and with the aid of the following primers:
SB210 (34 mer) (SEQ ID NO 23)
   5' AAATTTTCTGCAGATGGCGACAACAGCCATGAGG 3' and
SB211 (35 mer) (SEQ ID NO 24)
   5' TTAAGGATCCTCATTTACTAAAGGAAAGATTGTTG 3'.

The amplification product, having a size of 1739 bp, is digested with the enzymes PstI and BamHI and cloned into the vector pVR1012 (Example 2) previously digested with PstI and BamHI, thus generating the plasmid pSB 107, having a size of about 6583 bp.

The F gene of the BRSV virus encodes a protein of 574 amino acids.

### 4.1.2 pSB108: F gene (Δ[TM-Cter] form) cloned into the vector pVR1012

The truncated form of the F gene of the Snook strain of BRSV is amplified by RT-PCR using the viral RNA as template and with the aid of the following primers:
SB210 (SEQ ID NO 23) and
SB212 (39 mer) (SEQ ID NO 25)
   5' AATTTTGGATCCTCATGTGGTGGATTTTCCTACATCTAC 3'.

The amplification product (1581 bp) is digested with the enzymes PstI and BamHI and cloned into the vector pVR1012 (Example 2) previously digested with PstI and BamHI, generating the plasmid pSB108, having a size of about 6430 bp.

The truncated form of the F gene encodes a glycoprotein of 523 amino acids, containing the extracellular domain of the BRSV F glycoprotein.

### 4.1.3 pSB114: F gene (tPA Δ[TM-Cter] form) cloned into the vector pAB110

The tPA Δ[TM-Cter] form of the F gene of the BRSV Snook strain is amplified by RT-PCR using the viral RNA as template and with the aid of the following primers:
SB212 (SEQ ID NO 25) and
SB220 (38 mer) (SEQ ID NO 26)
   5' AAAATTCACGTGAACATAACAGAAGAATTTTATCAATC 3'.

The amplification product (1516 bp) is digested with the enzymes PmlI and BglII and cloned into the vector pAB110 (Example 2) previously digested with PmlI and BglII, generating the plasmid pSB114, having a size of about 6572 bp.

The tPA Δ[TM-Cter] form of the F gene encodes a glycoprotein of 535 amino acids, containing the extracellular domain of the BRSV F glycoprotein.

### 4.1.4 pPB449: F gene (large deletion Δ[TM-Cter] form) cloned into the vector pVR1012

The truncated form of the F gene of the Snook strain of BRSV is amplified by RT-PCR using the viral RNA as template and with the aid of the following primers:
SB210 (SEQ ID NO 23) and
FC129 (39 mer) (SEQ ID NO 57)
   5' AATTTTGGATCCTCAGATTCCACGATTTTTATTAGAAGC 3'.

The amplification product (1305 bp) is digested with the enzymes PstI and BamHI and cloned into the vector pVR1012 (Example 2) previously digested with PstI and BamHI, generating the plasmid pPB449, having a size of about 6150 bp.

The truncated form of the F gene encodes a glycoprotein of 431 amino acids, containing the extracellular domain of the BRSV F glycoprotein.

### 4.2 Plasmids encoding the various forms of BRSV-G

In the case of the BRSV G protein (type II glycoprotein), the signal sequence and the transmembrane sequence are indistinguishable, requiring the addition of a signal sequence upstream of the sequence corresponding to the extracellular domain during the deletion of the transmembrane domain.

The plasmid pAB110 (Example 2) is used for the construction of the plasmids containing the truncated forms of the gene encoding the BRSV G protein.

### 4.2.1 pSB109: G gene (native form) cloned into the vector pVR1012

The G gene of the BRSV Snook strain is amplified by RT-PCR using the viral RNA as template and with the aid of the following primers:
SB213 (32 mer) (SEQ ID NO 27)
   5'ACGCGTCGACATGTCCAACCATACCCATCATC 3' and
SB214 (38 mer) (SEQ ID NO 28)
   5' TTAAAATCTAGATTAGATCTGTGTAGTTGATTGATTTG 3'.

The amplification product (784 bp) is digested with enzymes SalI and XbaI and cloned into the vector pVR1012 (Example 2) previously digested with SalI and XbaI, generating the plasmid pSB109, having a size of about 5661 bp.

The BRSV G gene encodes a glycoprotein of 257 amino acids.

### 4.2.2 pSB110: G gene (tPA Δ[TM-Cter] form) cloned into the vector pAB110

The truncated form of the G gene of the BRSV Snook strain is amplified by RT-PCR using the viral RNA as template and with the aid of the following primers:
SB215 (33 mer) (SEQ ID NO 29)
   5' TTTTAAGGATCCGCTAAAGCCAAGCCCACATCC 3' and
SB216 (33 mer) (SEQ ID NO 30)
   5' TTAAAATCTAGATTAGATCTGTGTAGTTGATTG 3'.

The amplification product (666 bp) is digested with the enzymes BamHI and XbaI and cloned into the vector pAB110 (Example 2) previously digested with BamHI and XbaI, generating the plasmid pSB110, having a size of about 5660 bp.

The tPA Δ[TM-Cter] form of the BRSV virus G gene encodes a glycoprotein of 218 amino acids, containing the extracellular domain of the G glycoprotein, but preceded by the signal sequence of the tissue plasminogen activator.

### 4.3 pFC123: N gene (native form) cloned into the vector pVR1012

The N gene of BRSV is amplified by RT-PCR using the viral RNA as template and with the aid of the following primers:
FC 130 (34 mer) (SEQ ID NO 58)
   5' AAATTTTGTCGACATGGCTCTTAGCAAGGTCAAA 3' and
FC131 (35 mer) (SEQ ID NO 59)
   5' TTAAGGATCCTCACAGTTCCACATCATTGTCTTTG 3'.

The amplification product, having a size of 1199 bp, is digested with the enzymes SalI and BamHI and cloned into the vector pVR1012 (Example 2) previously digested with SalI and BamHI, thus generating the plasmid pFC123, having a size of about 6057 bp.

The N gene of the BRSV virus encodes a nucleocapsid protein of 391 amino acids.

### Example 5: Plasmids encoding the various forms of the bovine viral diarrhea virus type 1 (BVD-1) antigens

The genes encoding the E0 (glycoprotein of 48 kDa or gp48) and E2 (gp53) antigens of the type 1 BVDV viruses are obtained by RT-PCR from the viral RNA of the Osloss strain (L. De Moerlooze et al. J. Gen. Virol. 1993, 74, 1433-1438; A. Renard et al., DNA, 1985, 4, 439-438; A. Renard et al. Ann. Rech. Vet., 1987, 18, 121-125). The NADL (ATCC VR-534) or New York (ATCC VR-524) strains may also be used.

### 5.1 Plasmids encoding the various forms of E0 of the BVDV type 1 Osloss strain

### 5.1.1 pLF1028: E0 gene (native form) cloned into the vector pVR1012

The complementary DNA (cDNA) of the E0 gene of the Osloss strain is synthesized from the corresponding viral RNA with the aid of the primer LF051 and amplified by the PCR reaction with the aid of the following oligonucleotide pair:
LF050 (36 mer) (SEQ ID NO 31)
   5' CATACCGTCGACATGAAGAAACTAGAGAAAGCCCTG 3' and
LF051 (40 mer) (SEQ ID NO 32)
   5' CATACCGGATCCTCAGGCTGCATATGCCCCAAACCATGTC 3'.

The DNA fragment of about 765 bp obtained by digesting the PCR product with SalI and BamHI is ligated with a fragment of 4866 bp resulting from the digestion of pVR1012 (Example 2) with SalI and BamHI in order to generate the plasmid pLF1028 (about 5636 bp). The E0 gene of BVDV-1 strain Osloss encodes a protein of 252 amino acids.

An ATG codon is introduced into the sequence of the oligonucleotide LF050 so as to allow the initiation of the translation of the corresponding recombinant E0 polypeptide.

### 5.1.2 pLF1029: E0 gene, (β-globin tPA-E0) form cloned into the vector pLF999.

The E0 gene is synthesized by a PCR reaction from the pLF1028 template (Example 5.1.1) and with the aid of the following oligonucleotide pair:
LF052 (39 mer) (SEQ ID NO 33)
   5' CATGACGCGGCCGCTATGAAGAAACTAGAGAAAGCCCTG 3' and
LF053 (40 mer) (SEQ ID NO 34)
   5' CATGACAGATCTTTAGGCTGCATATGCCCCAAACCATGTC 3'.

The DNA fragment of about 770 bp obtained by digesting the PCR product with NotI and BglII is ligated with a fragment of 5642 bp resulting from the digestion of pLF999 (Example 2) with NotI and BglII in order to generate the plasmid pLF1029 (about 6417 bp).

The E0 gene of BVDV-1 strain Osloss thus modified (β-globin tPA-E0) encodes a protein of 283 amino acids.

### 5.2 Plasmids encoding the various forms of E2 of the BVDV type 1 Osloss strain

### 5.2.1 pLF1020: E2 gene (native form) cloned into the vector pVR1012

The cDNA of the E2 gene of the Osloss strain is synthesized from the corresponding viral RNA with the aid of the primer LF040 and amplified by a PCR reaction with the aid of the following oligonucleotide pair:
LF039 (33 mer) (SEQ ID NO 35)
   5' CATGACGTCGACATGACGACTACTGCATTCCTG 3' and
LF040 (36 mer) (SEQ ID NO 36)
   5' CATGACAGATCTTCAACGTCCCGAGGTCATTTGTTC 3'.

The DNA fragment of 1235 bp obtained by digesting the PCR product with SalI and BglII is ligated with a fragment of 4860 bp resulting from the digestion of pVR1012 (Example 2) with SalI and BglII in order to generate the plasmid pLF1020 (about 6100 pb).

The E2 gene of BVDV-1 strain Osloss encodes a protein of 409 amino acids.

An ATG codon is introduced into the sequence of the oligonucleotide LF039 so as to allow the initiation of the translation of the corresponding recombinant E2 polypeptide.

### 5.2.2 pLF1021: E2 gene, (β-globin tPA-E2 Δ[TM+Cter]) form cloned into the vector pLF999.

The E2 gene deleted for its transmembrane and carboxy-terminal domains is synthesized by a PCR reaction from the pLF1020 template (Example 5.2.1) and with the aid of the following oligonucleotide pair:
LF041 (36 mer) (SEQ ID NO 37)
   5' CATGACGCGGCCGCTATGACGACTACTGCATTCCTG 3' and
LF042 (35 mer) (SEQ ID NO 38)
   5' CATGACAGATCTCAAGCGAAGTAATCCCGGTGGTG 3.

The DNA fragment of 1132 bp obtained by digesting the PCR product with NotI and BglII is ligated with a fragment of 5642 bp resulting from the digestion of pLF999 (Example 2) with NotI and BglII in order to generate the plasmid pLF1021 (about 6779 bp).

The E2 gene of BVDV-1 strain Osloss thus modified (β-globin tPA-E2 Δ[TM+Cter]) encodes a protein of 404 amino acids.

### Example 6: Plasmids encoding the various forms of the bovine viral diarrhea virus type 2 (BVDV-2) antigens

The genes encoding the E2 antigen (gp53) of the BVDV type 2 viruses are obtained by RT-PCR from the viral RNA of the strain 890 (J.F. Ridpath and S.R. Bolin, Virology, 1995, 212, 36-46). The strain Q140 can also be used and may be obtained from the Quebec Ministry of Agriculture, Fisheries and Food, Armand-Frappier Institute (P. Tijssen et al., Virology, 1996, 217, 356-361). The strains 1373 and 296 may also be used (J.F. Ridpath, BVDV Research Project, National Animal Disease Center, 2300 Dayton Avenue, Ames, USA).

### 6.1 Plasmids encoding the various forms of E2 of the type 2 - 890 strain

### 6.1.1. pLF1022: E2 gene (native form) cloned into the vector pVR1012

The cDNA of the E2 gene of the strain 890 is synthesized from the corresponding viral RNA with the aid of the primer LF044 amplified by a PCR reaction with the aid of the following oligonucleotide pair:
LF043 (36 mer) (SEQ ID NO 39)
   5' ACTGTATCTAGAATGACCACCACAGCTTTCCTAATC 3' and
LF044 (39 mer) (SEQ ID NO 40)
   5' ACTGTAAGATCTTTAAGTATTCACTCCAGCACCCATAGC 3'.

The DNA fragment of about 1240 bp obtained by digesting the PCR product with XbaI and BglII is ligated with a fragment of 4891 bp resulting from the digestion of pVR1012 (Example 2) with XbaI and BglII in order to generate the plasmid pLF1022 (about 6136 bp).

The E2 gene of BVDV-2 strain 890 encodes a protein of 410 amino acids.

An ATG codon is introduced into the sequence of the oligonucleotide LF043 so as to allow the initiation of the translation of the corresponding recombinant E2 polypeptide.

### 6.1.2 pLF1023: E2 gene, (β-globin tPA-E2 Δ[TM+Cter]) form, cloned into the vector pLF999

The E2 gene deleted for its transmembrane and carboxy-terminal domains is synthesized by a PCR reaction from the pLF1022 template (Example 6.2.1) and with the aid of the following oligonucleotide pair:
LF045 (41 mer) (SEQ ID NO 41)
   5' CATGACGCGGCCGCCCTATGACCACCACAGCTTTCCTAATC 3' and
LF046 (36 mer) (SEQ ID NO 42)
   5' CATGACAGATCTTTATATGAACTCTGAGAAGTAGTC 3'.

The DNA fragment of about 1140 bp obtained by digesting the PCR product with NotI and BglII is ligated with a fragment of 5642 bp resulting from the digestion of pLF999 (Example 2) with NotI and BglII in order to generate the plasmid pLF1023 (about 6787 bp).

The E2 gene of BVDV-2 strain 890 thus modified (β-globin tPA-E2 Δ[TM+Cter]) encodes a protein of 405 amino acids.

### 6.2 Plasmids encoding the various forms of E0 of the type 2 - 890 strain

### 6.2.1 pLF 1030: E0 gene (native form) cloned into the vector pVR1012

The cDNA of the E0 gene of the 890 strain is synthesized from the corresponding viral RNA with the aid of the LF065 primer and amplified by a PCR reaction with the aid of the following oligonucleotide pair:
LF064 (39 mer) (SEQ ID NO 43)
   5' CATACCGTCGACATGAGAAAGAAATTGGAGAAGGCACTG 3' and
LF065 (39 mer) (SEQ ID NO 44)
   5' CATACCGGATCCTCATGCTGCATGAGCACCAAACCATGC 3'.

The DNA fragment of about 768 bp obtained by digesting the PCR product with SalI and BamHI is ligated with a fragment of 4866 bp resulting from the digestion of pVR1012 (Example 2) with SalI and BamHI in order to generate the plasmid pLF1030 (about 5639 bp). The E0 gene of BVDV-2 strain 890 encodes a protein of 253 amino acids.

An ATG codon is introduced into the sequence of the oligonucleotide LF064 so as to allow the initiation of the translation of the corresponding recombinant E0 polypeptide.

### 6.2.2 pLF1031: E0 gene, (β-globin tPA-E0) form, cloned into the vector pLF999.

The E0 gene is synthesized by a PCR reaction from the pLF1030 template (Example 6.2.1.) and with the aid of the following oligonucleotide pair:
LF066 (42 mer) (SEQ ID NO 45)
   5' CATGACGCGGCCGCTATGAGAAAGAAATTGGAGAAGGCACTG 3' and
LF067 (39 mer) (SEQ ID NO 46)
   5' CATACCAGATCTTCATGCTGCATGAGCACCAAACCATGC 3'.

The DNA fragment of about 770 bp obtained by digesting the PCR product with NotI and BglII is ligated with a fragment of 5642 bp resulting from the digestion of pLF999 (Example 2) with NotI and BglII in order to generate the plasmid pLF1031 (about 6417 bp).

The E0 gene of BVDV-2 strain 890 thus modified (β-globin tPA-E0) encodes a protein of 283 amino acids.

### Example 7: Plasmids encoding the various forms of the bovine parainfluenza virus type 3 (bPI-3) antigens

The genes encoding the hemagglutinin-neuraminidase (HN) and fusion (F) antigens of the bPI-3 virus are obtained by RT-PCR from the viral RNA of the Reisinger SF-4 strain (accessible from ATCC under the number VR-281).

### 7.1 Plasmids encoding the various forms of HN of the bPI-3 SF-4 strain

### 7.1.1 pLF1024: HN gene (native form) cloned into the vector pVR1012

The cDNA of the HN gene of the SF-4 strain is synthesized from the corresponding viral RNA with the aid of the primer LF048 and amplified by a PCR reaction with the aid of the following oligonucleotide pair:
LF047 (39 mer) (SEQ ID NO 47)
   5' CATATCGTCGACATGGAATATTGGAAACACACAAACAGC 3' and
LF048 (38 mer) (SEQ ID NO 48)
   5' CATGACGATATCTAGCTGCAGTTTTTCGGAACTTCTGT 3'.

The DNA fragment of 1726 bp obtained by digesting the PCR product with SalI and EcoRV is ligated with a fragment of 4896 bp resulting from the digestion of pVR1012 (Example 2) with SalI and EcoRV in order to generate the plasmid pLF1024 (about 6619 bp).

The bPI-3 HN gene encodes a protein of 572 amino acids.

### 7.1.2 pLF1025: HN gene, (β-globin tPA-E2 Δ[TM]) form, cloned into the vector pLF999

The HN gene deleted for its transmembrane domain is synthesized by a PCR reaction from the pLF1024 template (Example 7.1.1) with the aid of the following oligonucleotide pair:
LF058 (33 mer) (SEQ ID NO 49)
   5' CATACTGCGGCCGCTTTAATTCAAGAGAACAAT 3' and
LF059 (35 mer) (SEQ ID NO 50)
   5' CATATCGATATCTAGCTGCAGTTTTTCGGAACTTC 3'.

The DNA fragment of 1566 bp obtained by digesting the PCR product with NotI and EcoRV is ligated with a fragment of 5663 bp resulting from the digestion of pLF999 (Example 2) with NotI and EcoRV in order to generate the plasmid pLF1025 (about 7229 bp).

The bPI-3 HN gene thus modified (β-globin tPA-E2 Δ[TM]) encodes a protein of 548 amino acids.

### 7.2 Plasmids encoding the various forms of F of the bPI-3 SF-4 strain

### 7.2.1 pLF1026: F gene (native form) cloned into the vector pVR1012

The cDNA of the F gene of strain SF-4 is synthesized from the corresponding viral RNA with the aid of the primer LF061 and amplified by a PCR reaction with the aid of the following oligonucleotide pair:
LF060 (36 mer) (SEQ ID NO 51)
   5' CATATCGTCGACATGATCATCACAAACACAATCATA 3' and
LF061 (36 mer) (SEQ ID NO 52)
   5' CATGACCAGATCTTATTGTCTATTTGTCAGTATATA 3'.

The DNA fragment of 1628 bp obtained by digesting the PCR product with SalI and BglII is ligated with a fragment of 4860 bp resulting from the digestion of pVR1012 (Example 2) with SalI and BglII in order to generate the plasmid pLF1026 (about 6488 bp).

The bPI-3 F gene encodes a protein of 550 amino acids.

### 7.2.2 pLF1027: F gene, (β-globin tPA-F Δ[TM+Cter]) form, cloned into the vector pLF999

The F gene deleted for its transmembrane and C-terminal domains is synthesized by a PCR reaction from the pLR1026 template (Example 7.2.1) and with the aid of the following oligonucleotide pair:
LF062 (42 mer) (SEQ ID NO 53)
   5' CATACTGCGGCCGCTCAAATAGACATAACAAAACTGCAACGT 3' and
LF063 (41 mer) (SEQ ID NO 54)
   5' CATATCGATATCTATGCACTAGATTGATACCAACTTCCAAC 3'.

The DNA fragment of 1434 bp obtained by digesting the PCR product with NotI and EcoRV is ligated with a fragment of 5663 bp resulting from the digestion of pLF999 (Example 2) with NotI and EcoRV in order to generate the plasmid pLF1027 (about 7097 bp).

The bPI-3 F gene thus modified (β-globin tPA-F Δ[TM+Cter]) encodes a protein of 504 amino acids.

### Example 8: Plasmid encoding bovine GM-CSF

The cDNA of the bovine GM-CSF gene is synthesized from the cellular RNA of bovine blood mononucleated cells with the aid of the primer LF065 and amplified by a PCR reaction with the aid of the following oligonucleotide pair:
LF054 (36 mer) (SEQ ID NO 55)
   5' CATATCGTCGACATGTGGCTGCAGAACCTGCTTCTC 3' and
LF055 (34 mer) (SEQ ID NO 56)
   5' CATGACCAGATCTTCACTTCTGGGCTGGTTCCCA 3'.

The DNA fragment of 437 bp obtained by digesting the PCR product with SalI and BglII is ligated with a fragment of 4860 bp resulting from the digestion of pVR1012 (Example 2) with SalI and BglII in order to generate the plasmid pLF1032 (about 5297 bp). The bovine GM-CSF gene encodes a protein of 143 amino acids.

### Example 9: Formulation of the vaccinal plasmids

The DNA solution containing one or more plasmids according to Examples 3 to 8 is concentrated by ethanolic precipitation as described in Sambrook *et al.* (1989). The DNA pellet is taken up in a 0.9% NaCl solution so as to obtain a concentration of 1 mg/ml. A 0.75 mM DMRIE-DOPE solution is prepared by taking up a lyophilisate of DMRIE-DOPE with an appropriate volume of sterile H₂O.

The formation of the plasmid DNA-lipid complexes is achieved by diluting, in equal parts, the 0.75 mM DMRIE-DOPE solution with the DNA solution at 1 mg/ml in 0.9% NaCl. The DNA solution is gradually introduced, with the aid of a seamed 26G needle, along the wall of the vial containing the cationic lipid solution so as to avoid the formation of foam. Gentle shaking is carried out as soon as the two solutions have been mixed. A composition comprising 0.375 mM of DMRIE-DOPE and 500 µg/ml of plasmid is finally obtained.

It is desirable for all the solutions used to be at room temperature for all the operations described above. The DNA/DMRIE-DOPE complex formation is allowed to take place at room temperature for 30 minutes before immunizing the animals.

### Example 10: Immunization of bovines against BHV-1

12 bovines are randomized into 3 groups of 4 s.

Group 1 constitutes the control animal group.

A mixture of vaccinal plasmids pPB281 (encoding BHV-1 gB in a Δ[TM-Cter] form, Example 3.1.2), pPB292 (encoding BHV-1 gC in a Δ[TM-Cter] form, Example 3.2.2) and pPB284 (encoding BHV-1 gD in a Δ[TM-Cter] form, Example 3.3.2) is administered to the animals of Group 2.

The same mixture as that in Group 2, but formulated with DMRIE-DOPE as is described in Example 15, is administered to the animals of Group 3.

An injection of 10 ml, by the intramuscular route, is performed on each bovine with the aid of syringes equipped with needle, and is repeated 21 days later. The total mass of each plasmid used during each immunization is 1500 µg.

Persons skilled in the art possess the necessary competence to adjust the volume or the concentration according to the plasmid dose required.

Monitoring of the serological response induced by the two mixtures of vaccine plasmids expressing the BHV-1 gB, gC and gD antigens is carried out over a period of 35 days after the first vaccination.

The results are presented in the table which follows:

| Plasmids | Formulation | Antigens | Dose | SN at D28 | SN at D35 |
|---|---|---|---|---|---|
| Control | --- | --- | --- | 0.2 +/- 0.0 | 0.2 +/-0.0 |
| pPB281 | --- | gB Δ[TM-Cter] | 1500 µg | 1.0+/-0.5 | 1.2 +/- 0.8 |
| pPB292 | | gC Δ[TM-Cter] | 1500 µg | | |
| pPB294 | | gD Δ[TM-Cter] | 1500 µg | | |
| pPB281 | DMRIE-DOPE | gB Δ[TM-Cter] | 1500 µg | 2.1 +/- 0.6 | 2.7 +/- 0.6 |
| pPB292 | | gC Δ[TM-Cter] | 1500 µg | | |
| pPB294 | | gD Δ[TM-Cter] | 1500 µg | | |

### Example 11: Prime-boost immunization of bovines against BRSV

The vaccinal plasmids, encoding BRSV F in a large deletion Δ[TM-Cter] form and in a Δ[TM-Cter] form and encoding BRSV N, were concentrated by ethanolic precipitation as described in Sambrook *et al.* (1989). The DNA pellet was taken up in a 1.8% NaCl solution so as to obtain a concentration of 1.6 mg/ml. A 1.2 mM DMRIE-DOPE solution was prepared by taking up a lyophilisate of DMRlE-DOPE with an appropriate volume of sterile H₂O.

The formation of the plasmid DNA-lipid complexes was achieved by mixing, in equal parts, the 1.2 mM DMRIE-DOPE solution with the DNA solution at 1.6 mg/ml in 1.8% NaCl. The DNA solution was gradually introduced, with the aid of a seamed 26G needle, along the wall of the vial containing the cationic lipid solution so as to avoid the formation of foam. Gentle shaking was carried out as soon as the two solutions have been mixed. A composition comprising 0.6 mM of DMRIE-DOPE and 800 µg/ml of plasmid was finally obtained.

It is desirable for all the solutions used to be at room temperature for all the operations described above. The DNA/DMRIE-DOPE complex formation is allowed to take place at room temperature for 30 minutes before immunizing the animals.

BRSV (strain 375, deposited before the American Type Culture Collection (ATCC) under the accession number # VR-1339) was inactivated with β-propiolactone and formulated with Carbopol® 974P (Noveon Inc.) (20% v/v, 15 g/L Carbopol solution) to have a 3mg/ml final concentration of Carbopol.

20 bovines, 3 to 4 weeks old, were randomized into 4 groups of 5 animals.

Group 1 constitutes the control animal group (without any vaccination).

For the priming immunization, DNA vaccine formulated with DMRIE-DOPE was administered to the animals of Group 2 and Group 3 by the intramuscular route, 2 ml per dose, with a syringe and a needle. The total quantity of plasmids used for the immunization was 1600 µg. Inactivated vaccine formulated with Carbopol was administered to the animals of Group 4 by the intramuscular route, 5 ml per dose, with a syringe and a needle.

28 days later, a boost immunization was done. DNA vaccine formulated with DMRIE-DOPE was administered to the animals of Group 2 by the intramuscular route, 2 ml per dose, with a syringe and a needle. The total quantity of plasmids used for the immunization was 1600 µg. Inactivated vaccine formulated with Carbopol was administered to the animals of Group 3 and Group 4 by the intramuscular route, 5 ml per dose, with a syringe and a needle.

All the animals were challenged on day 193 with pathogenic BRSV (Snook strain; Taylor G. et al., J Gen Virol 1998, 79(7), 1759-67) at about 4.5 log10 CCID₅₀/ml. 10 ml of the inoculum suspension were inoculated intra-nasally to each of the calves by spray for a total infectious dose per calve of approximately 10^{5.5} CCID₅₀.

Monitoring of the rectal temperatures, respiratory rates, clinical scores, lung lesion scores, viral excretions and memory BRSV-specific IFNγ+ T cell response was carried out over a period of 10 days after the challenge.

The rectal temperatures results are presented in °C in the Figure 3.

All calves presented hyperthermia after challenge. Group 3 (DNA/inactivated) presented an average temperature peak earlier in time than the other group and rectal temperature were significantly lower (p=0.001) as compared to the control for the D4-D10 period.

The respiratory rates (breathing per minute) results are presented in the Figure 4.

Group 3 presented on average a lower and earlier respiratory rate peak than the other groups. For this group, a lower respiratory rate was observed (p=0.06) when compared to the controls.

The clinical scores results are scored as followed:

| Clinical sign | 0 | 1 | 2 |
|---|---|---|---|
| Prostation | No | Yes | - |
| Anorexia | None | Partial | Total |
| Coughing | None | occasionnaly | Repeted |
| Dyspnea | None | Moderate | Hard |

The score for mortality or for euthanasia for ethical reason is 8.

The clinical scores results are presented in the Figure 5.

All calves presented moderate to severe hyperpnoea after challenge. All calves were at least prostrated or partially anorectic one days during the challenge phase. There were considerable variation in the severity of signs, even within groups. For example, in the group 4, one animal was severely affected (deceased on D8) and its global clinical score (GSS = total score of the challenge phase) account for more than 50% of the total GSS for the group. The situation is even more striking in the group 3. Group where GSS of one calve represent nearly 2/3 of the total GSS for the group 3.

All calves (found dead or euthanazied) were necropsied. A macroscopic examination of the deep respiratory apparatus was performed and the dorsal and the ventral sides of the lung were observed for lung lesions. Size of lesions was estimated for each pulmonary lobe (both sides), as a percentage of lobe affected (surface affected/total surface), to calculate a lobe score as follow:
Lobe score= (lobe balance index) × (dorsal score + ventral score)/2
The lobe balance index represents the relative importance of pulmonary lobes and is:
For the right cranial lobe and the medium cranial lobe: 0.11
For the right medium caudal lobe: 0.07
For the right caudal lobe: 0.35
For the left cranial lobe: 0.05
For the left medium lobe: 0.06
For the left caudal lobe: 0.32
And for the azygos lobe: 0.04.

The lung lesion score of each calve was determined by addition of all lobe scores.

The lung lesion scores results are presented in the Figure 6.

Of particular interest, in the group 3, 4/5 calves had less than 7.5 % lung lesions whereas the remaining calve had almost 100 % lung lesions.

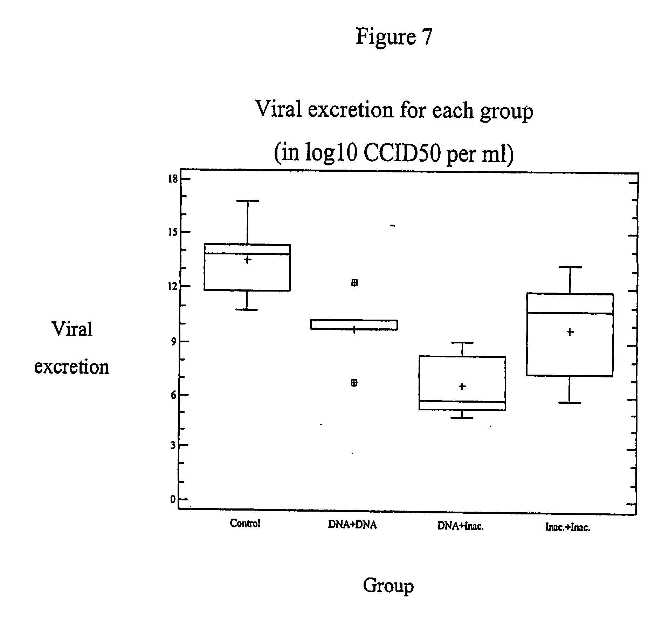

The nasal swabs viral excretion results are presented in the Figure 7.

BRSV challenge strain was detected in animals of all groups, however at a variable level, controls having the highest level of excretion. As compared to controls, the total excretion (sum of daily excretion title in log₁₀) was significantly reduced in the group 3 only.

*In vivo*-primed bovine peripheral blood mononuclear cells (PBMCs) were taken from blood samples collected at day 36 and 92 following vaccination and 10 days post-challenge.

Day 36 PBMCs were re-stimulated ex vivo with autologous dendritic cells infected either with a recombinant poxvirus expressing the F protein of BRSV or with the parental poxvirus as control.

Day 92 and 10 days post-challenge PBMCs were re-stimulated ex vivo directly with live BRSV grown on VERO cells (strain 375, from about 6.0 log10 CCID₅₀/ml to about 6.4 log10 CCID₅₀/ml). As control, PBMCs were mock infected by a VERO cell lysate.

The frequencies of antigen-specific T cells was determined by the number of IFNγ-secreting cells in a quantitative enzyme-linked immune spot (ELISPOT) assay (Laval F. et al., Vet. Immunol. Immunopathol., 2002, 90(3-4), 191-201). Using this approach the vaccines strategies can be rank for their ability to prime viral-specific IFNγ(+)T cells.

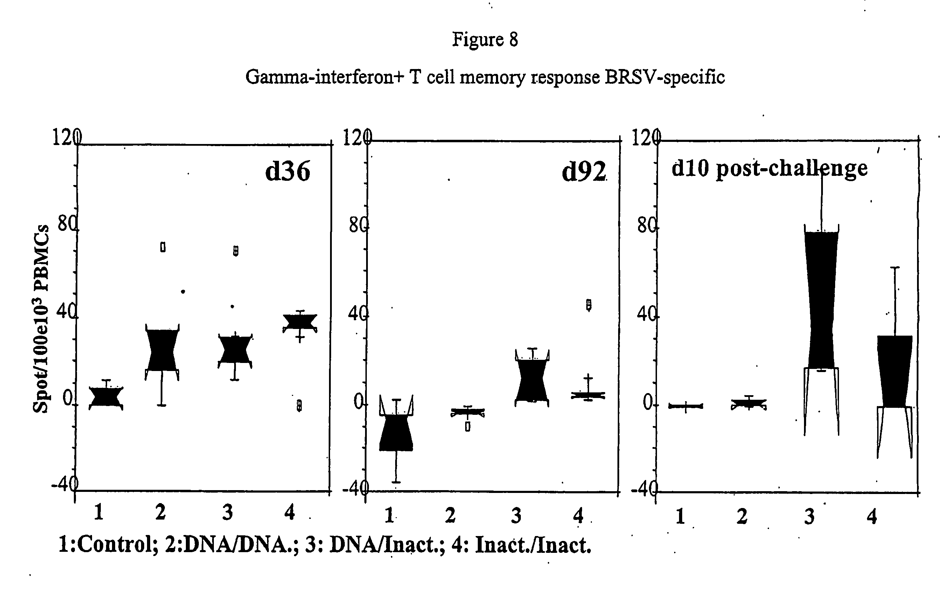

The memory BRSV-specific IFNγ+ T cell responses are presented in the Figure 8.

In contrast to the day 36 time point, where the results translated into the secondary effector T cell response, day 92 analysis revealed the memory T cell response (2 months following the second shot). It appeared that calves with a good level of memory specific T cell response were found mainly in the group 3 (calves vaccinated with the DNA/inactivated prime-boost).

Interestingly, the calves of the group 3 which consistently showed a memory BRSV-specific IFNγ+ T cell response, whatever the time point analysed, are the calves where a significant protection was observed.

The description will be further described by the following numbered paragraphs:
1. Use of a plasmid containing and expressing *in vivo* in a bovine such as cattle at least one immunogen from a bovine pathogen, selected from BRSV, bPI-3, BHV-1 and BVDV, for the preparation of a DNA vaccine intended to induce an immune response into young bovines such as calves which have or may have maternal antibodies against said bovine pathogen.
2. Use according to paragraph 1, wherein the DNA vaccine is intended to be administered to the young animal or bovine from calving up to and including 12 weeks of age, such as from calving up to and including 6 weeks of age, such as from calving up to and including 4 weeks of age, and especially from calving up to and including 3 weeks of age.
3. Use according to paragraph 1 or 2, wherein said DNA vaccine is intended to induce a priming immune response, such as with a IFNγ+ memory T cell response specific for the expressed immunogen, which priming immune response can be boosted by a subsequent administration of an inactivated vaccine or a live recombinant vaccine comprising a viral vector, such as a live recombinant poxvirus, containing and expressing *in vivo* at least the same immunogen(s) than that expressed by the DNA vaccine.
4. Use of a bovine pathogenic agent, selected from BRSV, bPI-3, BHV-1 and BVDV, for the preparation of a priming DNA vaccine comprising a plasmid containing and expressing *in vivo* in a bovine such as cattle at least one immunogen from said pathogenic agent, and for the preparation of a second vaccine comprising said pathogenic agent under an inactivated form, wherein the DNA vaccine is intended to be administered to a bovine such as cattle first, such as to a young calve which have or may have maternal antibodies against said bovine pathogen, and the inactivated vaccine is intended to be administered after the DNA vaccine and to the same bovine such as cattle such as the young calve, to boost the immune response against said immunogen.
5. Use according to paragraph 4, wherein the DNA vaccine is intended to induce in the bovine such as cattle, such as the calve, an immune response against said immunogen(s), such as the gamma+ interferon memory T cell response specific for the expressed immunogen.
6. Use according to paragraph 4 or 5, wherein the DNA vaccine is intended to be administered to the young animal or bovine from calving up to and including 12 weeks of age, such as from calving up to and including 6 weeks of age, such as from calving up to and including 4 weeks of age, and especially from calving up to and including 3 weeks of age.
7. Use according to paragraph 4, 5 or 6, wherein the inactivated vaccine is intended to be administered from about 2 weeks to about 5 months after the priming administration, such as from about 3 to 6 weeks after, and such as about 4 weeks after the DNA vaccine was administered.
8. Use of a nucleotide sequence coding for at least one immunogen from a bovine pathogenic agent, selected from BRSV, bPI-3, BHV-1 and BVDV, for the preparation of a priming DNA vaccine comprising a plasmid containing and expressing *in vivo* said immunogen and for the preparation of a second vaccine comprising a live recombinant viral vector, such as a live recombinant poxvirus, containing and expressing *in vivo* at least said immunogen(s), wherein the DNA vaccine is intended to be administered to a bovine first, such as to a young calve which have or may have maternal antibodies against said bovine pathogen, and the viral vector-based vaccine is intended to be administered after the DNA vaccine and to the same bovine such as the calve, to boost the immune response against said immunogen.
9. Use according to paragraph 8, wherein the DNA vaccine is intended to induce a DNA vaccine induced immune response against said immunogen(s), such as the IFN_{γ}+ memory T cell response specific for the expressed immunogen.
10. Use according to paragraph 8 or 9, wherein the DNA vaccine is intended to be administered to the young animal or bovine from calving up to and including 12 weeks of age, such as from calving up to and including 6 weeks of age, such as from calving up to and including 4 weeks of age, and especially from calving up to and including 3 weeks of age.
11. Use according to paragraph 8, 9 or 10, wherein the live viral vector-based vaccine is intended to be administered from about 2 weeks to about 5 months after the priming administration, such as from about 3 to 6 weeks after, and such as about 4 weeks after the DNA vaccine was administered.
12. Use of a bovine pathogenic agent, selected from BRSV, bPI-3, BHV-1 and BVDV, to prepare an inactivated vaccine intended to vaccinate a bovine against said pathogenic agent, wherein the bovine, such as a young calve which have or may have maternal antibodies against said bovine pathogen, has previously been immunized with a DNA vaccine expressing *in* vivo at least one immunogen from the same pathogenic agent and has developed a specific priming DNA vaccine induced immune response, such as the IFN_{γ}+ memory T cell response specific for the expressed immunogen.
13. Use according to paragraph 12, wherein the inactivated vaccine is intended to be administered from about 2 weeks to about 5 months, such as from about 3 to 6 weeks, and such as about 4 weeks after the bovine was administered with the DNA vaccine.
14. Use of a recombinant viral vector, such as a poxvirus vector, comprising and expressing *in vivo* at least one nucleotide sequence coding for at least one immunogen from a bovine pathogenic agent, selected from BRSV, bPI-3, BHV-1 and BVDV, to prepare a live recombinant vaccine intended to vaccinate a bovine against the pathogenic agent, wherein the bovine, such as a young calve which have or may have maternal antibodies against said bovine pathogen, has previously been immunized with a DNA vaccine expressing *in vivo* at least the same immunogen(s), has developed a priming DNA vaccine induced immune response, such as the IFN_{γ}+ memory T cell response specific for the expressed immunogen.
15. Use according to paragraph 14, wherein the live viral vector-based vaccine is intended to be administered about 2 weeks to about 5 months, such as from about 3 to 6 weeks, and such as about 4 weeks after the bovine was administered with the DNA vaccine.
16. Prime-boost vaccination method of a bovine such as cattle against at least one bovine pathogen, wherein the bovine or cattle is first administered with a priming DNA vaccine comprising and expressing *in vivo* an immunogen from said pathogen, and then is boosted with a second type of vaccine presenting the same immunogen.
17. Method according to paragraph 16, wherein the boost is done with an inactivated vaccine.
18. Method according to paragraph 16, wherein the boost is done with a vaccine comprising a recombinant live viral vector, such as a recombinant poxvirus, comprising and expressing *in vivo* the said immunogen.
19. Method according to any one of paragraph 16 to 18, wherein the DNA vaccine is administered to a young calve that can have maternal antibodies against the pathogenic agent against which immunization or vaccination is directed.
20. Method according to any one of paragraph 16 to 19, wherein the DNA vaccine is administered to the young animal or bovine from calving up to and including 12 weeks of age, such as from calving up to and including 6 weeks of age, such as from calving up to and including 4 weeks of age, and especially from calving up to and including 3 weeks of age.
21. Method according to any one of paragraphs 16 to 20, wherein the boost administration is administered from about 2 weeks to about 5 months after the priming administration, such as from about 3 to 6 weeks after, and such as about 4 weeks after.
22. Method according to any one of paragraphs 16 to 21, wherein a second administration of the boost vaccine is done, such as when the bovine or calves are transferred to the finishing units.
23. The prime-boost vaccination method of a cattle against at least one bovine pathogen, which comprises administering first to a bovine such as cattle a priming DNA vaccine comprising a nucleotide sequence encoding and expressing *in vivo* an immunogen from said pathogen, and then administering a second type of vaccine presenting the same immunogen, e.g., an inactivated, attenuated, subunit, or recombinant live viral vector vaccine, advantageously an inactivated vaccine or a recombinant live viral vector, such as a recombinant poxvirus, comprising and expressing *in vivo* the said immunogen. The method can be applied to a young calve that can have maternal antibodies against the pathogenic agent against which immunization or vaccination is directed.

## Claims

1. A kit for performing a prime-boost vaccination method against a bovine pathogen comprising:
(a) a first; priming vaccine, wherein the first, priming vaccine comprises a DNA vaccine comprising nucleic acid molecule(s) encoding and expressing *in vivo* in a bovine at least one immunogen from the bovine pathogen, wherein the immunogen is BRSV F or BRSV N or an epitope thereof or combinations thereof, and
(b) a second, boosting vaccine wherein the second, boosting vaccine against the bovine pathogen is different to the first, priming vaccine, but contains or expresses at least one immunogen of the bovine pathogen which is the same immunogen of the bovine pathogen expressed by the first, priming vaccine, wherein the second, boosting vaccine is an inactivated pathogen wherein the pathogen is bovine respiratory syncytial virus (BRSV);
wherein (a) and (b) are in separate containers, optionally with instructions for administration or use; and
wherein the bovine pathogen is bovine respiratory syncytial virus (BRSV).

2. The kit of claim 1, wherein (a) and (b) are in separate containers in the same package.

3. Use of a bovine pathogenic agent, wherein the bovine pathogenic agent is BRSV, for the preparation of a priming DNA vaccine comprising a plasmid containing and expressing *in vivo* in a bovine at least one immunogen from said bovine pathogenic agent wherein the immunogen is BRSV F or BRSV N or an epitope thereof or combinations thereof, and for the preparation of a second vaccine comprising said bovine pathogenic agent under an inactivated form wherein the bovine pathogenic agent is bovine respiratory syncytial virus (BRSV), wherein the priming DNA vaccine is intended to be administered to a bovine first, and the inactivated vaccine is administered after the priming DNA vaccine and to the same bovine to boost the immune response against said immunogen.

4. Use according to claim 3, wherein the priming DNA vaccine is intended to induce in the bovine an immune response against said immunogen(s).

5. Use according to claim 4 wherein said immune response against said immunogen(s) is a gamma+ interferon memory T cell response specific for the expressed immunogen.

6. Use according to any one of claims 3 to 5, wherein the priming DNA vaccine is intended to be administered to a young bovine from calving up to and including 12 weeks of age.

7. Use according to any one of claims 3 to 6, wherein the second vaccine is intended to be administered from 2 weeks to 5 months after the priming DNA vaccine is administered.

8. Use of a bovine pathogenic agent, wherein the bovine pathogenic agent is BRSV, to prepare an inactivated vaccine for vaccinating a bovine against said pathogenic agent, wherein the bovine, has previously been immunized with a DNA vaccine expressing *in vivo* at least one immunogen from the same pathogenic agent, wherein the immunogen is BRSV F or BRSV N or an epitope thereof or combinations thereof, and the bovine has developed a specific priming DNA vaccine induced immune response.

9. Use according to claim 8 wherein said immune response is an IFNγ+ memory T cell response specific for the expressed immunogen.

10. Use according to claim 8 or 9, wherein the inactivated vaccine is intended to be administered from 2 weeks to 5 months after the bovine was administered with the DNA vaccine.

11. Use according to any one of claims 3 to 10, wherein the bovine is a young calf which has maternal antibodies against said bovine pathogen.

12. The kit of claim 1 or 2 wherein said DNA vaccine contains DMRIE.

13. The use according to any one of claims 3 to 11 wherein said DNA vaccine contains DMRIE.

14. A prime-boost vaccination for use in the treatment and/or prevention of BRSV;
wherein a priming DNA vaccine comprises a plasmid containing and expressing *in vivo* in a bovine at least one immunogen from a bovine pathogenic agent wherein the bovine pathogenic agent is BRSV and wherein the immunogen is BRSV F or BRSV N or an epitope thereof or combinations thereof; and wherein a booster vaccine comprises a bovine pathogenic agent under an inactivated form wherein the bovine pathogenic agent is bovine respiratory syncytial virus (BRSV);
wherein the priming DNA vaccine is intended to be administered to a bovine first, and the inactivated vaccine is intended to be administered after the priming DNA vaccine and to the same bovine to boost the immune response against said immunogen.

15. An inactivated vaccine for use in the treatment and/or prevention of BRSV;
wherein the inactivated vaccine comprises a bovine pathogenic agent, wherein the bovine pathogenic agent is BRSV, and wherein the pathogenic agent is inactivated;
wherein the inactivated vaccine is intended to be administered to a bovine that has previously been immunized with a DNA vaccine expressing *in vivo* at least one immunogen from the same pathogenic agent, wherein the immunogen is BRSV F or BRSV N or an epitope thereof or combinations thereof, and the bovine has developed a specific priming DNA vaccine induced immune response.

## Patentansprüche

1. Kit zur Durchführung eines Prime-Boost-Impfverfahrens gegen einen Rindererreger, umfassend:
(a) einen ersten Prime-Impfstoff, wobei der erste Prime-Impfstoff einen DNA-Impfstoff umfasst, der (ein) Nucleinsäure-Molekül(e) umfasst, das/die mindestens ein Immunogen von dem Rindererreger codiert/codieren und *in vivo* in einem Rind exprimiert/exprimieren, wobei das Immunogen BRSV F oder BRSV N oder ein Epitop davon oder Kombinationen davon ist, und
(b) einen zweiten Boost-Impfstoff, wobei der zweite Boost-Impfstoff gegen den Rindererreger sich von dem ersten Prime-Impfstoff unterscheidet, jedoch mindestens ein Immunogen des Rindererregers enthält oder exprimiert, das das gleiche Immunogen des Rindererregers ist, das von dem ersten Prime-Impfstoff exprimiert wird, wobei der zweite Boost-Impfstoff ein inaktivierter Erreger ist, wobei der Erreger das bovine respiratorische Syncytialvirus (BRSV) ist;
wobei (a) und (b) in separaten Behältern sind, gegebenenfalls mit Anweisungen für die Verabreichung oder Verwendung; und
wobei der Rindererreger das bovine respiratorische Syncytialvirus (BRSV) ist.

2. Kit gemäß Anspruch 1, wobei (a) und (b) in separaten Behältern in der gleichen Verpackung sind.

3. Verwendung eines Rindererregeragens, wobei das Rindererregeragens BRSV ist, für die Herstellung eines Prime-DNA-Impfstoffs, der ein Plasmid umfasst, das mindestens ein Immunogen von dem Rindererregeragens enthält und *in vivo* in einem Rind exprimiert, wobei das Immunogen BRSV F oder BRSV N oder ein Epitop davon oder Kombinationen davon ist, und für die Herstellung eines zweiten Impfstoffs, der das Rindererregeragens in einer inaktivierten Form enthält, wobei das Rindererregeragens das bovine respiratorische Syncytialvirus (BRSV) ist, wobei der Prime-DNA-Impfstoff einem Rind zuerst verabreicht werden soll, und der inaktivierte Impfstoff nach dem Prime-DNA-Impfstoff und dem selben Rind verabreicht wird, um die Immunantwort gegen das Immunogen zu steigern.

4. Verwendung gemäß Anspruch 3, wobei der Prime-DNA-Impfstoff in dem Rind eine Immunantwort gegen das/die Immunogen(e) induzieren soll.

5. Verwendung gemäß Anspruch 4, wobei die Immunantwort gegen das/die Immunogen(e) eine Gamma-Interferon+-Gedächtnis-T-Zellantwort ist, die spezifisch für das exprimierte Immunogen ist.

6. Verwendung gemäß einem der Ansprüche 3 bis 5, wobei der Prime-DNA-Impfstoff einem jungen Rind im Zeitraum vom Abkalben bis einschließlich 12 Wochen danach verabreicht werden soll.

7. Verwendung gemäß einem der Ansprüche 3 bis 6, wobei der zweite Impfstoff 2 Wochen bis 5 Monate nachdem der Prime-DNA-Impfstoff verabreicht wurde, verabreicht werden soll.

8. Verwendung eines Rindererregeragens, wobei das Rindererregeragens BRSV ist, für die Erstellung eines inaktivierten Impfstoffs zur Impfung eines Rinds gegen das Erregeragens, wobei das Rind zuvor mit einem DNA-Impfstoff immunisiert wurde, welcher *in vivo* mindestens ein Immunogen von dem gleichen Erreger exprimiert, wobei das Immunogen BRSV F oder BRSV N oder ein Epitop davon oder Kombinationen davon ist, und das Rind eine spezifische Immunantwort entwickelt hat, die durch den Prime-DNA-Impfstoff induziert wurde.

9. Verwendung gemäß Anspruch 8, wobei die Immunantwort eine IFNγ+ Gedächtnis-T-Zellantwort ist, die spezifisch für das exprimierte Immunogen ist.

10. Verwendung gemäß Anspruch 8 oder 9, wobei der inaktivierte Impfstoff 2 Wochen bis 5 Monate, nachdem dem Rind der DNA-Impfstoff verabreicht wurde, verabreicht werden soll.

11. Verwendung gemäß einem der Ansprüche 3 bis 10, wobei das Rind ein junges Kalb ist, das mütterliche Antikörper gegen den Rindererreger hat.

12. Kit gemäß Anspruch 1 oder 2, wobei der DNA-Impfstoff DMRIE enthält.

13. Verwendung gemäß einem der Ansprüche 3 bis 11, wobei der DNA-Impfstoff DMRIE enthält.

14. Prime-Boost-Impfung zur Verwendung für die Behandlung und/oder Prävention von BRSV;
wobei ein Prime-DNA-Impfstoff ein Plasmid umfasst, das mindestens ein Immunogen von einem Rindererregeragens enthält und *in vivo* in einem Rind exprimiert, wobei das Rindererregeragens BRSV ist und wobei das Immunogen BRSV F oder BRSV N oder ein Epitop davon oder Kombinationen davon ist; und
wobei ein Boost-Impfstoff ein Rindererregeragens in einer inaktivierten Form umfasst, wobei das Rindererregeragens das bovine respiratorische Syncytialvirus (BRSV) ist;
wobei der Prime-DNA-Impfstoff einem Rind zuerst verabreicht werden soll und der inaktivierte Impfstoff nach dem Prime-DNA-Impfstoff und dem selben Rind verabreicht werden soll, um die Immunantwort gegen das Immunogen zu steigern.

15. Inaktivierter Impfstoff zur Verwendung für die Behandlung und/oder Prävention von BRSV;
wobei der inaktivierte Impfstoff ein Rindererregeragens umfasst, wobei der Rindererreger BRSV ist, und wobei das Erregeragens inaktiviert ist;
wobei der inaktivierte Impfstoff einem Rind verabreicht werden soll, das zuvor mit einem DNA-Impfstoff immunisiert wurde, welcher *in vivo* mindestens ein Immunogen von dem selben Erregeragens exprimiert, wobei das Immunogen BRSV F oder BRSV N oder ein Epitop davon oder Kombinationen davon ist, und das Rind eine spezifische Immunantwort entwickelt hat, die von dem Prime-DNA-Impfstoff induziert wurde.

## Revendications

1. Kit pour mettre en oeuvre un procédé de vaccination de sensibilisation-renforcement contre un pathogène bovin comprenant :
(a) un premier vaccin, de sensibilisation, où le premier vaccin, de sensibilisation comprend un vaccin à ADN comprenant une ou des molécules d'acide nucléique codant et exprimant *in vivo* chez un bovin au moins un immunogène provenant du pathogène bovin, où l'immunogène est BRSV F ou BRSV N ou un épitope de ceux-ci ou des combinaisons de ceux-ci,
et
(b) un second vaccin, de renforcement, où le second vaccin, de renforcement contre le pathogène bovin est différent du premier vaccin, de sensibilisation mais contient ou exprime au moins un immunogène du pathogène bovin qui est le même immunogène du pathogène bovin exprimé par le premier vaccin, de sensibilisation, où le second vaccin, de renforcement est un pathogène inactivé où le pathogène est le virus syncytial respiratoire bovin (BRSV) ;
où (a) et (b) sont dans des récipients séparés, éventuellement avec des instructions pour l'administration ou l'utilisation ; et
ou le pathogène bovin est le virus syncytial respiratoire bovin (BRSV).

2. Kit selon la revendication 1 où (a) et (b) sont dans des récipients séparés dans le même emballage.

3. Utilisation d'un agent pathogène bovin, où l'agent pathogène bovin est BRSV, pour la préparation d'un vaccin à ADN de sensibilisation comprenant un plasmide contenant et exprimant *in vivo* dans un bovin au moins un immunogène provenant dudit agent pathogène bovin où l'immunogène est BRSV F ou BRSV N ou un épitope de ceux-ci ou des combinaisons de ceux-ci, et pour la préparation d'un second vaccin comprenant ledit agent pathogène bovin sous une forme inactivée où l'agent pathogène bovin est le virus syncytial respiratoire bovin (BRSV), où le vaccin à ADN de sensibilisation est destiné à être administré à un bovin d'abord, et le vaccin inactivé est administré après le vaccin à ADN de sensibilisation et au même bovin pour renforcer la réponse immunitaire contre ledit immunogène.

4. Utilisation selon la revendication 3, où le vaccin à ADN de sensibilisation est destiné à induire chez le bovin une réponse immunitaire contre ledit ou lesdits immunogènes.

5. Utilisation selon la revendication 4 où ladite une réponse immunitaire contre ledit ou lesdits immunogènes est une réponse à cellules T à mémoire interféron gamma+ spécifique de l'immunogène exprimé.

6. Utilisation selon l'une quelconque des revendications 3 à 5 où le vaccin à ADN de sensibilisation est destiné à être administré à un jeune bovin depuis le vêlage jusques et y compris l'âge de 12 semaines.

7. Utilisation selon l'une quelconque des revendications 3 à 6 où le second vaccin est destiné à être administré de 2 semaines à 5 mois après que le vaccin à ADN de sensibilisation soit administré.

8. Utilisation d'un agent pathogène bovin, où l'agent pathogène bovin est BRSV, pour préparer un vaccin inactivé pour la vaccination d'un bovin contre ledit agent pathogène, où le bovin a été immunisé auparavant avec un vaccin à ADN exprimant *in vivo* au moins un immunogène provenant du même agent pathogène, où l'immunogène est BRSV F ou BRSV N ou un épitope de ceux-ci ou des combinaisons de ceux-ci, et le bovin a développé une réponse immunitaire induite par le vaccin à ADN de sensibilisation spécifique.

9. Utilisation selon la revendication 8 où ladite réponse immunitaire est une réponse à cellules T à mémoire IFNγ+ spécifique de l'immunogène exprimé.

10. Utilisation selon la revendication 8 ou 9 où le vaccin inactivé est destiné à être administré de 2 semaines à 5 mois après que le vaccin à ADN ait été administré au bovin.

11. Utilisation selon l'une quelconque des revendications 3 à 10 où le bovin est un jeune veau qui a des anticorps maternels contre ledit pathogène bovin.

12. Kit selon la revendication 1 ou 2 où ledit vaccin à ADN contient du DMRIE.

13. Utilisation selon l'une quelconque des revendications 3 à 11 où ledit vaccin à ADN contient du DMRIE.

14. Vaccination de sensibilisation-renforcement destinée à être utilisée dans le traitement et/ou la prévention du BRSV ;
où un vaccin à ADN de sensibilisation comprend un plasmide contenant et exprimant *in vivo* chez un bovin au moins un immunogène provenant d'un agent pathogène bovin où l'agent pathogène bovin est BRSV et où l'immunogène est BRSV F ou BRSV N ou un épitope de ceux-ci ou des combinaisons de ceux-ci ; et où un vaccin de renforcement comprend un agent pathogène bovin sous une forme inactivée où l'agent pathogène bovin est le virus syncytial respiratoire bovin (BRSV) ;
où le vaccin à ADN de sensibilisation est destiné à être administré à un bovin d'abord, et le vaccin inactivé est destiné à être administré après le vaccin à ADN de sensibilisation et au même bovin pour renforcer la réponse immunitaire contre ledit immunogène.

15. Vaccin inactivé destiné à être utilisé dans le traitement et/ou la prévention du BRSV ;
où le vaccin inactivé comprend un agent pathogène bovin, où l'agent pathogène bovin est BRSV, et où l'agent pathogène est inactivé ;
où le vaccin inactivé est destiné à être administré à un bovin qui a été immunisé auparavant avec un vaccin à ADN exprimant *in vivo* au moins un immunogène provenant du même agent pathogène, où l'immunogène est BRSV F ou BRSV N ou un épitope de ceux-ci ou des combinaisons de ceux-ci, et le bovin a développé une réponse immunitaire induite par le vaccin à ADN de sensibilisation spécifique.
